(19) Europäisches Patentamt
European Patent Office
Office européen des brevets

(11) Publication number : **0 372 816 B1**

(12) **EUROPEAN PATENT SPECIFICATION**

(45) Date of publication of patent specification : **29.07.92 Bulletin 92/31**

(51) Int. Cl.$^5$ : **C07D 339/08,** A01N 43/28, C07F 7/08

(21) Application number : **89312432.1**

(22) Date of filing : **29.11.89**

# (54) Novel heterocyclic pesticidal compounds.

(30) Priority : **30.11.88 GB 8827885**

(43) Date of publication of application : **13.06.90 Bulletin 90/24**

(45) Publication of the grant of the patent : **29.07.92 Bulletin 92/31**

(84) Designated Contracting States : **AT BE CH DE ES FR GB GR IT LI LU NL SE**

(56) References cited :
**EP-A- 0 294 228**
**DE-A- 3 437 935**

(73) Proprietor : **THE WELLCOME FOUNDATION LIMITED**
**Unicorn House 160 Euston Road**
**London NW1 2BP (GB)**

Proprietor : **THE REGENTS OF THE UNIVERSITY OF CALIFORNIA**
**300 Lakeside Drive, 22nd Floor, Office of the President**
**Oakland, California 94612-3550 (US)**

(72) Inventor : **Larkin, John Patrick**
**The Wellcome Foundation Limited**
**Berkhamsted Hertfordshire (GB)**
Inventor : **Parkin, Donald**
**The Wellcome Foundation Limited**
**Berkhamsted Hertfordshire (GB)**
Inventor : **Palmer, Christopher John**
**37 Clapgate Lane**
**Ipswich Suffolk (GB)**
Inventor : **Casida, John Edward**
**1570 La Vereda Road**
**Berkeley California 94708 (US)**

(74) Representative : **Rollins, Anthony John et al**
**Group Patents & Agreements The Wellcome Research Laboratories Langley Court**
**Beckenham Kent BR3 3BS (GB)**

**EP 0 372 816 B1**

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Jouve, 18, rue Saint-Denis, 75001 PARIS

## Description

The present invention is concerned with a method of controlling pests such as arthropods, e.g. insects and acarine pests, and helminths, e.g. nematodes, by contacting the pests with novel pesticides. The invention is also concerned with the novel pesticides used for controlling the pests and processes for making such pesticides.

This invention was made with United States Government support under Grant No. P01 ES 00049 from the National Institutes of Health to The University of California. The United States Government has certain rights in this invention.

Current classes of pesticides effectively control some but not all pest species. It is also desirable to have new classes of pesticides since pests tend to develop resistance to any one pesticide, or sometimes to any one class of pesticide, after they have been selected with or exposed to such pesticides over a period of time.

Certain 2,5-dialkylsubstituted dithianes have been investigated as liquid crystal materials (see for example Mol. Cryst. Liq. Cryst., 131. 101) but no pesticidal activity has been reported for such compounds. 5-Alkyl-2-substituted dithianes are disclosed as having insecticidal activity in European Patent Application No. 0294229.

It has been discovered that a class of novel 2,5-disubstituted dithianes has pesticidal activity.

Accordingly, the present invention provides a compound of the formula (I):

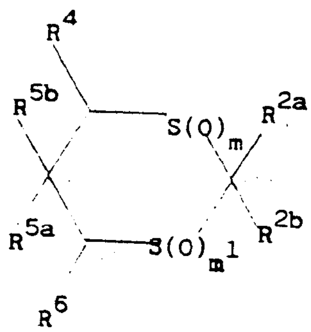

(I)

which contains between 9 and 27 carbon atoms, and wherein m and $m^1$ are independently selected from 0, 1 and 2; $R^{2a}$ is hydrogen, methyl, or ethyl; $R^{2b}$ is ethynyl or contains between 3 and 18 carbon atoms and is a group $R^7$, wherein $R^7$ is a $C_{1-13}$ non-aromatic hydrocarbyl group, optionally substituted by a cyano or $C_{1-4}$ carbalkoxy group and/or by one or two hydroxy groups and/or by one to five halo atoms which are the same or different and/or by one to three groups $R^8$ which are the same or different and each contains one to four hetero atoms, which are the same or different and are chosen from oxygen, sulphur, nitrogen and silicon, 1 to 10 carbon atoms and optionally 1 to 6 fluoro or chloro atoms or $R^{2b}$ is a 6-membered aromatic ring substituted by cyano and/or by one to three groups $R^{8a}$ and/or by a group -C≡CH, -C≡C-$R^{7a}$ or C≡C-halo and/or by one to five halo atoms and/or by one to three $C_{1-4}$ haloalkyl groups wherein $R^{7a}$ and $R^{8a}$ are groups $R^7$ and $R^8$ as hereinbefore defined; $R^4$ and $R^6$ are the same or different and are chosen from hydrogen, methyl, trifluoromethyl or cyano; $R^{5a}$ is a group

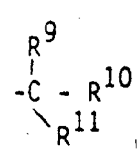

wherein $R^{11}$ is hydrogen, methyl, trifluoromethyl, iodo, fluoro, chloro or bromo, $R^9$ is methyl, ethyl, chloro, bromo, methoxy, cyano, nitro, methoxymethyl, $C_{1-4}$ carbalkoxy or trifluoromethyl, $R^{10}$ is chloro, methyl or trifluoromethyl, or the moiety

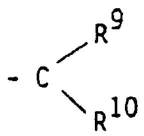

is a -C=$CR^{12}R^{13}$ group wherein $R^{12}$ and $R^{13}$ are both hydrogen, methyl, trifluoromethyl, fluoro, chloro or bromo, or $R^{12}$ is hydrogen and $R^{13}$ is fluoro, chloro or bromo, or the moiety

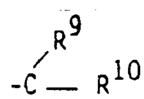

is a three or four membered ring,

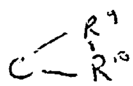

in which $R^9$ is oxygen or a group $CR^{14}R^{15}$ wherein the groups $R^{14}$ and $R^{15}$ are the same or different and each is hydrogen, fluoro, chloro or bromo or methyl or ethyl optionally substituted by 1 to 3 fluoro atoms, and in which $R^{10}$ is a group $CR^{14a}R^{15a}$ or a group $CR^{14a}R^{15a}CR^{16}R^{17}$ wherein $R^{14a}$,$R^{15a}$ are the same or different and each is hydrogen, fluoro, chloro or bromo or methyl or ethyl optionally substituted by 1 to 5 fluoro atoms, and $R^{16}$, and $R^{17}$ are the same or different and each is hydrogen, fluoro, chloro or bromo or methyl or ethyl optionally substituted by 1 to 5 fluoro atoms; and $R^{5b}$ is hydrogen or $C_{1-3}$ alkyl provided that $R^{2b}$ is not propyl or butyl and that $R^{5a}$ is not tertiary butyl or tertiary amyl.

By the term "halo" is meant fluoro, chloro, bromo or iodo.

By the term "non-aromatic hydrocarbyl" group is meant an alkyl, alkenyl or alkynyl group (including a cyclic alkyl or alkenyl group optionally substituted by alkyl, alkenyl or alkynyl; and alkyl or alkenyl substituted by cyclic alkyl and alkenyl).

By the term "6-membered aromatic ring" is meant phenyl and heteroaromatic rings such as pyridyl.

$R^{2b}$ suitably contains between 3 and 12 carbon atoms. $R^{2b}$ is suitably a $C_{3-9}$ alkyl, alkenyl or alkynyl group, each of which may be optionally substituted by halo or a group $R^8$, or a substituted phenyl or substituted cyclohexyl group. The group $R^8$ is linked to the hydrocarbyl group or the aromatic ring via a hetero atom in $R^8$. Suitable substituents $R^8$ include alkoxy, alkenyloxy, alkynyloxy, alkoxyalkoxy, acyloxy, alkynyloximino, trialkylsilyl, haloalkoxy, haloalkenyloxy, haloalkynyloxy, alkyloximino, carbalkoxy, mono or di-substituted alkylamino groups or a group $-(O)_nS(O)_r(O)_tR^{18}$ wherein $R^{18}$ is a $C_{1-4}$ alkyl, alkenyl or alkynyl group each optionally substituted by up to 5 halo atoms, n and t are each 0 or 1, r is 0, 1 or 2, the sum of n,r and t being between 0 and 3. When a silyl group is present this is normally adjacent to an ethynyl group. Preferred substituents $R^8$ include alkoxy, alkoxyalkoxy, alkenyloxy, alkynyloxy, haloalkoxy, haloalkenyloxy and haloalkynyloxy. Suitably $R^7$ is substituted by up to two substituents $R^8$ and preferably $R^7$ is unsubstituted or contains one substituent $R^8$. Preferably there is only one silyl group present. The sulphur atoms present may be in an oxidised form if desired. Preferably there is a maximum of two sulphur atoms present in $R^{2b}$. Suitably there is a maximum of four and preferably a maximum of three oxygen atoms in $R^{2b}$. Preferably there is only one nitrogen atom present in $R^{2b}$.

In one suitable embodiment, $R^{2b}$ is a phenyl group substituted at the 3-,4- or 5-positions by one to three substituents each selected from halo, $C_{1-4}$ haloalkyl, $C_{1-4}$ haloalkoxy, $C_{1-4}$ haloalkylthio, cyano, or a group $(C{\equiv}C)_pR^{19}$ wherein p is 1 or 2 and $R^{19}$ is hydrogen, bromo, chloro, iodo or a group $S(O)_qR^{20}$ wherein q is 0, 1 or 2 and $R^{20}$ is trifluoromethyl, methyl or ethyl; or $R^{19}$ is an aliphatic group containing up to five carbon atoms optionally substituted by $C_{1-4}$ alkoxy, $C_{1-6}$ alkoxyalkoxy, $C_{1-8}$ acyloxy, halo or hydroxy or $R^{19}$ is a group $COR^{21}$ wherein $R^{21}$ is hydrogen, $C_{1-4}$ alkoxy, $C_{1-4}$ alkyl or a group $NR^{22}R^{23}$ wherein $R^{22}$ and $R^{23}$ are independently selected from hydrogen, methyl or ethyl; or $R^{19}$ is $SiR^{24}$, $R^{25}$, $R^{26}$ wherein $R^{24}$ and $R^{25}$ are the same or different and are each $C_{1-4}$ aliphatic groups and $R^{26}$ is a $C_{1-4}$ aliphatic group or phenyl provided that $R^{24}$, $R^{25}$ and $R^{26}$ do not contain more than 10 carbon atoms in total. The phenyl group is additionally optionally substituted at the 2- and/or 6-positions by fluoro or chloro. Suitably when the substituent is a group $(C{\equiv}C)_pR^{19}$, there is only one such substituent on the phenyl ring.

In one preferred embodiment $R^{2b}$ is phenyl substituted at the 3-, 4- or 5-positions by one to three substituents each selected from halo, cyano, $C_{1-4}$ haloalkyl or a group $C{\equiv}C\text{-}R^{27}$ where $R^{27}$ is hydrogen, methyl, or

ethyl each optionally substituted by hydroxy, methoxy, ethoxy, acetoxy; or $R^{27}$ is $C_{1-4}$ carbalkoxy, or a silyl group substituted by three $C_{1-4}$ alkyl groups. $R^{2b}$ is additionally optionally substituted at the 2- and/or 6- positions by fluoro or chloro.

In a second preferred embodiment $R^{2b}$ is a group -A(C≡C)Z, wherein A is a $C_{3-5}$ aliphatic chain optionally containing a double bond and/or an oxygen atom and/or a group S(O)q wherein q is 0, 1 or 2 optionally substituted by halo, $C_{1-4}$ alkyl, $C_{1-4}$ haloalkyl, $C_{1-4}$ carbalkoxy or cyano and Z is hydrogen, $C_{1-5}$ alkyl, $C_{1-3}$ alkoxymethyl or a group $SiR^{24},R^{25},R^{26}$ wherein $R^{24}$, $R^{25}$ and $R^{26}$ are as hereinbefore defined.

In a third preferred embodiment $R^{2b}$ is a group $-BZ^1$, wherein B is a group $-CH_2O-$ or $CH_2S(O)_q$ wherein q is 0, 1 or 2 or a $C_{2-3}$ aliphatic group each of which may be optionally substituted by one to three halo atoms and $Z^1$ is silyl substituted by three $C_{1-4}$ alkyl groups or $Z^1$ is a group

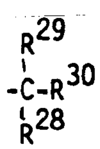

wherein $R^{28}$, $R^{29}$ and $R^{30}$ are the same or different and are each independently selected from halo, cyano, $C_{1-5}$ carbalkoxy, or a $C_{1-4}$ aliphatic group optionally substituted by halo,cyano, $C_{1-5}$ carbalkoxy, $C_{1-4}$ alkoxy or a group $S(O)_qR^{31}$ wherein q is 0, 1 or 2 and $R^{31}$ is $C_{1-4}$ alkyl, or $R^{28}$, $R^{29}$ and $R^{30}$ are selected from $C_{1-4}$ alkoxy or a group $S(O)_wR^{32}$ wherein w is 0, 1 or 2 and $R^{32}$ is $C_{1-4}$ alkyl optionally substituted by fluoro or $R^{28}$ and $R^{29}$ are linked to form a $C_{3-6}$ cycloalkyl ring, or one of $R^{28}$, $R^{29}$ and $R^{30}$ may be hydrogen.

By the term "aliphatic group" is meant an alkyl, alkenyl or alkynyl group.

Most suitably B is a group -C≡C- -CH=CH- or $-CH_2CH_2-$.

Preferably $Z^1$ is tertiary butyl, trichloromethyl or 2-methoxyprop-2-yl.

In a fourth preferred embodiment $R^{2b}$ is a group wherein Z is as hereinbefore defined:

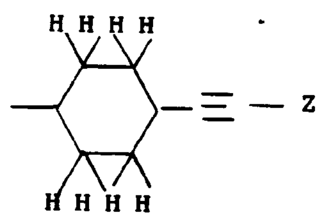

Preferably $R^{2a}$ is hydrogen or methyl.

Preferably $R^4$ and $R^6$ are hydrogen.

Suitably $R^{5a}$ is an isopropyl, isopropenyl cyclopropyl, methylcyclopropyl, cyclobutyl, 1-trifluoromethylethyl or 1,1-dimethyl-2,2,2-trifluoro ethyl group.

Preferably $R^{5a}$ is an isopropyl group.

A preferred group of compounds of the formula (I) is that in which $R^{2b}$ contains a -(C≡C)- fragment or terminates in a group $Z^1$ as hereinbefore defined or $R^{2b}$ is a para-bromophenyl group.

Some of the compounds of the formula (I) may exist in a number of stereoisomeric forms. The present invention encompasses both individual conformational and stereoisomers and mixtures thereof. The present invention also encompasses radiolabelled compounds of the formula (I), particularly those in which one carbon atom is $C^{14}$ or one or more hydrogen atoms are replaced by tritium.

Preferred compounds of the invention include:

cis/trans-2-(4-Bromophenyl)-5-isopropyl-1,3-dithiane.
cis/trans-5-Isopropyl-2-(4-trimethylsilylethynylphenyl)-1,3-dithiane.
cis/trans-2-(4-Ethynylphenyl)-5-isopropyl-1,3-dithiane.
trans-2-(4-Ethynylphenyl)-5-isopropyl-1,3-dithiane.
cis/trans-2-(4-Ethynylphenyl)-5-isopropyl-2-methyl-1,3-dithiane.
cis/trans-2-[4-(3-Acetoxyprop-1-ynyl)phenyl]-5-isopropyl-1,3-dithiane.
trans-2-[4-(3-Acetoxyprop-1-ynyl)phenyl]-5-isopropyl-1,3-dithiane.
trans-2-[4-(3-Benzoyloxyprop-1-ynyl)phenyl]-5-isopropyl-1,3-dithiane.
cis/trans-2-(trans-4-ethynylcyclohexyl)-5-isopropyl-1,3-dithiane.
cis/trans-2-(Hex-5-ynyl)-5-isopropyl-1,3-dithiane.

cis/trans-2-(trans-4-ethynylcyclohexyl)-5-isopropyl-1,3-dithiane oxide.
trans-2-(4-Bromophenyl)-5-isopropyl-1,3-dithiane 1,3-dioxide.
2(e)-(4-Ethynylphenyl)-5(e)-isopropyl-1,3-dithiane 1(e)-oxide.
cis-2(e)-(4-Ethynylphenyl)-5(a)-isopropyl-1,3-dithiane.
trans-2(e)-(trans-4(e)-Ethynylcyclohexyl)-5(e)-isopropyl-1,3-dithiane 1(e)-oxide.
trans-2(e)-[(E)-Hex-1-en-5-ynyl)-5(e)-isopropyl-1,3-dithiane.
cis/trans-5-Isopropyl-2-methyl-2-(3,3,3-trichlopropyl)-1,3-dithiane
cis/trans-2(e)-4-Ethynylphenyl)-5-(1-trifluoromethylethyl)-1,3-dithiane
trans-2(e)-(4-Ethynylphenyl)-5(e)-(1-trifluoromethylethyl)-1,3-dithiane.
trans-2(e)-(trans-4(e)-Ethynylcyclohexyl)-5(e)-1-trifluoromethylethyl)-1,3-dithiane.
cis/trans-5(e)-(1,1-Dimethyl-2,2,2-trifluoroethyl)-2-(4-trimethylsilylethynylphenyl)-1,3-dithiane.
trans-5(e)-(1,1-Dimethyl-2,2,2-trifluoroethyl)-2(e)-4-ethynylphenyl)1,3-dithiane.
cis/trans-5(e)-(1,1-Dimethyl-2,2,2-trifluoroethyl)-2-(4-ethynylphenyl)-1,3-dithiane.
cis/trans-5-Cyclopropyl-2-(cis/trans-4-ethynylcyclohexyl)-1,3-dithiane.
trans-5(e)-Cyclopropyl-2(e)-(4-ethynylphenyl)-1,3-dithiane.
trans-5-Cyclobutyl-2-(4-ethynylphenyl)-1,3-dithiane.
trans-2-(4-Ethynylphenyl)-5-(1-methylcyclopropyl)-1,3-dithiane.
cis/trans-5-(1-Methylpropyl)-2-(4-ethynylphenyl)-1,3-dithiane.
trans-5(e)-(1-Methylpropyl)-2(e)-(4-trimethylsilylethynylphenyl)-1,3-dithiane.

The present invention also provides for the preparation of the compounds of the formula (I) by methods derived from those known in the art for the preparation of analogous compounds. Thus, the compounds may be prepared by (i) the reaction of a compound of the formula (II):

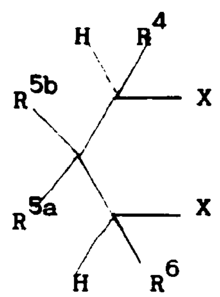

(II)

wherein X is SH with a suitable aldehyde or ketone of the formula

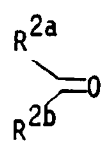

or a reactive derivative thereof, wherein $R^{2a}$, $R^{2b}$, $R^4$, $R^{5a}$, $R^{5b}$ and $R^6$ are as hereinbefore defined and, if required, thereafter oxidizing one or both of the ring sulphur atoms.

The reaction is suitably carried out in the presence of a catalyst or of a dehydrating agent in a non-polar solvent at a non-extreme temperature. Suitable catalysts include a dimethyl formamide/dimethyl sulphate catalyst and catalysts such as sulphonic acids or perfluorinated resins thereof or Lewis acids such as boron trifluoride etherate, or stannic chloride or concentrated formic acid which also serves as the reaction medium. Suitable solvents include hydrocarbons such as benzene, toluene or xylene or chlorinated hydrocarbons such as dichloromethane. The reaction is normally performed between 0° and 200° and conveniently between 20° and 120°.

Suitable reactive derivatives of aldehydes and ketones include acetals and ketals.

The compounds of the formula (II) may be prepared from the corresponding diols wherein X is hydroxy via the sulphonate derivatives (i.e., compounds of the formula (II) wherein X is a group $OSO_2R^{33}$ wherein $R^{33}$ is $C_{1-4}$ alkyl or para-tolyl) as outlined in Appendix 1. The preparation of the diols and their conversion to the corresponding dithiols can be carried out by methods known in the art for example as outlined in Appendices 1 and 2.

The aldehydes and ketones reacted with the dithiols of the formula (II) are either known in the literature or are prepared by literature methods, for example, the ethynylcyclohexylcarboxaldehydes are prepared as outlined in Appendix 3.

(ii) When $R^{2a}$ is hydrogen, the reaction of a dithiaborinane-dimethylsulphide complex of a compound of the formula (II) with a carboxylic acid

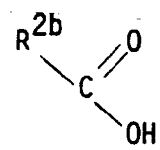

This reaction is carried out in the presence of a reducing agent such as stannous chloride in an inert solvent such as an ether, conveniently tetrahydrofuran, at a non-extreme temperature, for example between -20° and 100° and conveniently between 10° and 30°.

The dithiaborinane-dimethylsulphide complex is prepared from the corresponding dithiol by methods well known to those skilled in the art.

It is often convenient to prepare compounds of the formula (I) by interconversion from other compounds of the formula (I), for example:

(a) when it is required to prepare a compound of the formula (I) which contains an ethynyl group.

(i) by the reaction of the corresponding compound in which $R^{2b}$ is a 6-membered aromatic ring which contains iodo in place of $-C{\equiv}C-R^{34}$ with a compound $HC{\equiv}CR^{34}$ wherein $R^{34}$ is a group $R^7$ or $R^{19}$ as hereinbefore defined. This reaction is carried out in the presence of a suitable palladium catalyst well known to those skilled in the art for this type of reaction, for example bistriphenylphosphine palladium dichloride, and a catalytic amount of a cuprous halide, such as cuprous iodide. The reaction will normally be carried out in the presence of basic solvent such as diethylamine or triethylamine at a non-extreme temperature, for example between -50° and 100° and conveniently at room temperature. The starting material, i.e. the iodophenyl dithiane may be prepared as described above.

(ii) By the conversion of a group, for example a group $CH{=}C(hal)_2$ or $(hal)CH{=}CH_2$ wherein hal is chloro or bromo, into an ethynyl group.

The reaction is conveniently carried out by methods well known to those skilled in the art, for example when the group $-CH{=}C(hal)_2$ at about or below room temperature, for example between -70°C and 25°C, in an inert solvent, conveniently an ether such as tetrahydrofuran, in a base, for example n-butyl lithium.

(b) when it is desired to prepare a compound of the formula (I) from a compound of formula I which contains a group $-C{\equiv}C-H$, by reaction of the anion from such a compound with an alkylating or acylating agent hal $R^{7a}$, hal $R^{19}$, hal $R^{27}$ or halZ respectively, wherein hal is halogen and $R^{7a}$, $R^{19}$, $R^{27}$ or Z is other than hydrogen. This reaction is particularly suitable for the preparation of those compounds wherein $R^{7a}$, $R^{19}$, $R^{27}$ or Z is a $C_{1-4}$ alkyl group or a group $COR^{35}$ wherein $R^{35}$ is a $C_{1-4}$ alkoxy group. The reaction is normally carried out in the presence of a strong base, such as an alkyllithium conveniently butyllithium in an inert solvent, such as an ether, for example tetrahydrofuran, at a non-extreme temperature, for example between -50° and 50°C and conveniently between -10° and 30°. The starting material, e.g. the unsubstituted alkynylphenyl dithiane may be prepared as described above.

(c) when it is desired to prepare a compound of the formula (I) wherein $R^{19}$, $R^{27}$ or Z is hydrogen by the desilylation of a compound of the formula (I) wherein $R^{19}$, $R^{27}$ or Z is a tri-$C_{1-4}$ alkylsilyl group. This reaction may be carried out by methods well known to those skilled in the art, for example by reaction with tetrabutylammonium fluoride in an ether, such as tetrahydrofuran, at a non-extreme temperature, for example between 0° and 70°C and conveniently at room temperature.

(d) when it is required to convert a compound of the formula (I) wherein $R^{2a}$ is an axial hydrogen atom to the corresponding compound wherein $R^{2a}$ is an equatorial hydrogen atom by the addition of a strong base to the compound of the formula (I). The reaction is conveniently carried out in an inert solvent, conveniently an ether such as tetrahydro furan, at a non-extreme temperature, conveniently -50° to 50°C and conveniently at 0°C followed by quenching with water. If the reaction is carried out in the presence of an alkylating agent, such as methyl iodide, the corresponding equatorial alkylated compound is formed.

(e) when it is required to prepare a compound of the formula (I) wherein $R^{2b}$ contains a hydroxyalkyl group by the reduction of the corresponding compound containing an ester group. This reduction is conveniently carried out by a complex metal hydride such as lithium aluminium hydride in an inert solvent such as an ether, for example diethyl ether, at a non-extreme temperature, for example betweeen 0°C and 70°C and conveniently at room temperature.

(f) The compounds of the formula (I) may contain two or more sulphur atoms which may be oxidised if

required. Oxidations can be carred out by methods well known to those skilled in the art, for example using peracids such as peracetic acid from hydrogen peroxide and acetic acid, or 3-chloroperbenzoic acid in chloroform or dichloromethane, or using periodate such as tetrabutylammonium periodate in a halogenated hydrocarbon, for example chloroform at a non-extreme temperature, for example between 0° and 100°C and conveniently between 10° and 30°C.

When $R^{5a}$ contains halo atoms, it is preferred to prepare the compounds of the formula (I) via process (i)

The compounds of formula (I) may be used to control pests such as arthropods, e.g. insect and acarine pests, and helminths, e.g. nematodes. Thus, the present invention provides a method for the control of arthropods and/or helminths which comprises administering to the arthropod and/or helminth or to their environment an effective amount of a compound of the formula (I). The present invention also provides a method for the control of arthropod and/or helminth infestations of animals (including humans) and/or of plants (including trees) and/or stored products which comprises administering an effective amount of a compound of the formula (I). The present invention further provides for the compounds of the formula (I) for use in human and veterinary medicine, in public health control and in agriculture for the control of arthropod and/or helminth pests.

By the term "control" is meant the amelioration in air, water, soil or foliage of present or future deleterious effects of pests and includes killing adults, larvae and eggs, the inhibition of reproduction, the repellency and/or knockdown of pests, and any other influence on behaviour.

Compounds of formula (I) are of particular value in the protection of field, forage, plantation, glasshouse, orchard and vineyard crops, of ornamentals and of plantation and forest trees, for example, cereals (such as maize, wheat, rice, millet, oats, barley, sorghum), cotton, tobacco, vegetables and salads (such as beans, cole crops, cucurbits, lettuce, onions, tomatoes and peppers), field crops (such as potato, sugar beet, ground nuts, soyabean, oil seed rape), sugar cane, grassland and forage crops (such as lucerne), plantations (such as of tea, coffee, cocoa, banana, oil palm, coconut, rubber, spices), orchards and groves (such as of stone and pip fruit, citrus fruits, kiwifruit, avocado, mango, olives and walnuts), vineyards, ornamental plants, flowers and shrubs under glass and in gardens and parks, forest trees (both deciduous and evergreen) in forests, plantations and nurseries and plants grown for industrial or pharmaceutical purposes (such as the evening primrose).

They are also valuable in the protection of timber (standing, felled, converted, stored or structural) from attack by sawflies (e.g. Urocerus) or beetles (e.g. scolytids, platypodids, lyctids, bostrychids, cerambycids, anobiids) termites (e.g.Isoptera) or other damaging pests.

They have applications in the protection of stored products such as grains, fruits, nuts, spices and tobacco, whether whole, milled or compounded into products, from moth, beetle and mite attack. Also protected are stored animal products such as skins, hair, wool and feathers in natural or converted form (e.g. as carpets or textiles) from moth and beetle attack; also stored meat and fish from beetle, mite and fly attack.

Compounds of formula (I) are of value in the control of public health pests, for example cockroaches and ants.

Compounds of formula I are also of value in the control of arthropods or helminths which are injurious to, or spread or act as vectors of diseases in man and domestic animals, for example those hereinbefore mentioned, and more especially in the control of ticks, mites, lice, fleas, midges biting, nuisance and myiasis flies, mosquitos and hemiptrean bugs.

The compounds of Formula (I) may be used for such purposes by application of the compounds themselves or in diluted form in known fashion as a dip, spray, fog, lacquer, foam, dust, powder, aqueous suspension, paste, gel, cream, shampoo, grease, combustible solid, vapourising mat, combustible coil, bait, dietary supplement, wettable powder, granule, aerosol, emulsifiable concentrate, oil suspension, oil solution, pressure-pack, impregnated article, microcapsule, pour on formulation or other standard formulations well known to those skilled in the art. Sprays may be applied by hand or by means of a spray race or arch or by vehicle or aircraft mounted apparatus. The animal, soil, plant or other surface being treated may be saturated with the spray by means of high volume application or superficially coated with the spray by means of light or ultra low volume application. Dip concentrates are not applied per se, but diluted with water and the animals immersed in a dipping bath containing the dip wash. Aqueous suspensions may be applied in the same manner as sprays or dips. Dusts may be distributed by means of a powder applicator or, in the case of animals, incorporated in perforated bags attached to trees or rubbing bars. Pastes, shampoos and greases may be applied manually or distributed over the surface of an inert material, such as that against which animals rub and transfer the material to their skins. Pour-on formulations are dispensed as a unit of liquid of small volume on to the backs of animals such that all or most of the liquid is retained on the animals.

Compounds of Formula (I) may be prepared either as formulations ready for use on the animals, plants or surface or as formulations requiring dilution prior to application, but both types of formulation comprise a compound of Formula (I) in intimate admixture with one or more carriers or diluents. The carriers may be liquid,

solid or gaseous or comprise mixtures of such substances, and the compound of Formula (I) may be present in a concentration of from 0.025 to 99% w/v depending upon whether the formulation requires further dilution.

Dusts, powders and granules and other solid formulations comprise the compound of formula (I) in intimate admixture with a powdered solid inert carrier for example suitable clays, kaolin, bentonite, attapulgite, adsorbent carbon black, talc, mica, silica, chalk, gypsum, tricalcium phosphate, powdered cork, magnesium silicate, vegetable carriers, starch or a diatomaceous earth. Such solid formulations are generally prepared by impregnating the solid diluents with solutions of the compound of formula (I) in volatile solvents, evaporating the solvents and, if desired, grinding the products so as to obtain powders and, if desired, granulating, compacting or encapsulating the products.

Sprays of a compound of Formula (I) may comprise a solution in an organic solvent (e.g. those listed below) or an emulsion in water (dip wash or spray wash) prepared in the field from an emulsifiable concentrate (otherwise known as a water miscible oil) which may also be used for dipping purposes. The concentrate preferably comprises a mixture of the active ingredient, with or without an organic solvent and one or more emulsifiers. Solvents may be present within wide limits but preferably in an amount of from 0 to 99.5% w/v of the composition and may be selected from kerosene, ketones, alcohols, xylene, aromatic naphtha, water, mineral oil, aromatic and aliphatic esters, and other solvents known in the formulating art. The concentration of emulsifiers may be varied within wide limits but is preferably in the range of 5 to 25% w/v and the emulsifiers are conveniently nonionic surface active agents including polyoxyalkylene esters of alkyl phenols and polyoxyethylene derivatives of hexitol anhydrides and anionic surface active agents including Na lauryl sulphate, fatty alcohol ether sulphates, Na and Ca salts of alkyl aryl sulphonates and alkyl sulphosuccinates, soaps, lecithins, hydrolysed glues, etc.

Wettable powders comprise an inert solid carrier, one or more surface active agents, and optionally stabilisers and/or anti-oxidants.

Emulsifiable concentrates comprise emulsifying agents, and often an organic solvent, such as kerosene, ketones, alcohols, xylenes, aromatic naphtha, or other solvents known in the art.

Wettable powders and emulsifiable concentrates will normally contain from 0.5 to 99.5% by weight of the active ingredient, and are diluted, for example with water, before use.

Lacquers comprise a solution of the active ingredient in an organic solvent, together with a resin, and optionally a plasticiser.

Dip washes may be prepared not only from emulsifiable concentrates but also from wettable powders, soap based dips and aqueous suspensions comprising a compound of Formula (I) in intimate admixture with a dispersing agent and one or more surface active agents.

Aqueous suspensions of a compound of Formula (I) may comprise a suspension in water together with suspending, stabilizing or other agents. The suspensions or solutions may be applied per se or in a diluted form in known fashion.

Greases (or ointments) may be prepared from vegetable oils, synthetic esters of fatty acids or wool fat together with an inert base such as soft paraffin. A compound of Formula (I) is preferably distributed uniformly through the mixture in solution or suspension. Greases may also be made from emulsifiable concentrates by diluting them with an ointment base.

Pastes and shampoos are also semi-solid preparations in which a compound of Formula (I) may be present as an uniform dispersion in a suitable base such as soft or liquid paraffin or made on a non-greasy basis with glycerin, mucilage or a suitable soap. As greases, shampoos and pastes are usually applied without further dilution, they should contain the appropriate percentage of the compound of Formula (I) required for treatment.

Aerosol sprays may be prepared as a simple solution of the active ingredient in the aerosol propellant and co-solvent such as halogenated alkanes, propane, butane, dimethyl ether and the solvents referred to above, respectively. Pour-on formulations may be made as a solution or suspension of a compound of Formula (I) in a liquid medium. An avian or mammal host may also be protected against infestation of acarine ectoparasites by means of carrying a suitably-moulded, shaped plastics article impregnated with a compound of Formula (I). Such articles include impregnated collars, tags, bands, sheets and strips suitably attached to appropriate parts of the body. Suitably the plastics material is polyvinyl chloride (PVC).

The concentration of the compound of formula (I) to be applied to an animal, premises, other substrates or outdoor areas will vary according to the compound chosen, the interval between treatments, the nature of the formulation and the likely infestation, but in general 0.001 to 20.0% w/v and preferably 0.01 to 10% of the compound should be present in the applied formulation. The amount of the compound deposited will vary according to the compound chosen, the method of application, area of application, concentration of the compound in the applied formulation, factor by which the formulation is diluted and the nature of the formulation.

Undiluted formulations such as pour-on formulations in general will be applied at a concentration in the range from 0.1 to 20.0% w/w and preferably 0.1 to 10%. The amount of compound to be applied to stored products in general will lie in the range of from 0.1 to 20ppm. Space sprays may be applied to give an average initial concentration of 0.001 to 1 mg of compound of formula (I) per cubic metre of treated space.

Compounds of formula (I) are of use in the protection and treatment of plant species, in which case an effective insecticidal, acaricidal or nematocidal amount of the active ingredient is applied to the plant or the medium in which the plant is grown. The application rate will vary according to the compound chosen, the nature of the formulation, the mode of application, the plant species, the planting density and likely infestation and other like factors but in general, a suitable use rate for agricultural crops is in the range 0.001 to 3kg/Ha and preferably between 0.01 and 1kg/Ha. Typical formulations for agricultural use contain between 0.0001% and 50% of a compound of formula (I) and conveniently between 0.1 and 15% by weight of a compound of the formula (I).

Dusts, greases, pastes and aerosol formulations are usually applied in a random fashion as described above and concentrations of 0.001 to 20% w/v of a compound of Formula (I) in the applied formulation may be used.

The compounds of formula (I) have been found to have activity against the common housefly (Musca domestica). In addition, certain compounds of formula (I) have activity against other arthropod pests including Myzus persicae, Tetranychus urticae, Plutella xylostella, Culex ssp. Tribolium castaneum, Sitophilus granarius, Periplaneta americana and Blattella germanica. The compounds of formula (I) are thus useful in the control of arthropods e.g. insects and acarines in any environment where these constitute pests, e.g. in agriculture, in animal husbandry, in public health control and in domestic situations.

Insect pests include members of the orders Coleoptera (e.g. Anobium,Ceutorhynchus,Rhynchophorus, Cosmopolites, Lissorhoptrus, Meligethes, Hypothenemus, Hylesinus, Acalymma, Lema, Psylliodes, Leptinotarsa, Gonocephalum, Agriotes, Dermolepida, Heteronychus, Phaedon, Tribolium, Sitophilus, Diabrotica, Anthonomus or Anthrenus spp.), Lepidoptera (e.g. Ephestia, Mamestra, Earias, Pectinophora, Ostrinia, Trichoplusia, Pieris, Laphygma, Agrotis, Amathes, Wiseana, Tryporyza, Diatraea, Sporganothis, Cydia, Archips, Plutella, Chilo, Heliothis, Spodoptera or Tineola spp.), Diptera (e.g. Musca, Aedes, Anopheles, Culex, Glossina, Simulium, Stomoxys, Haematobia, Tabanus, Hydrotaea, Lucilia, Chrysomia, Callitroga, Dermatobia, Gasterophilus, Hypoderma, Hylemyia, Atherigona, Chlorops, Phytomyza, Ceratitis, Liriomyza and Melophagus spp.), Phthiraptera (Malophaga e.g. Damalina spp. and Anoplura e.g. Linognathus and Haematopinus spp.), Hemiptera (e.g. Aphis, Bemisia,Phorodon, Aeneolamia, Empoasca, Parkinsiella, Pyrilla, Aonidiella, Coccus, Pseudococcus, Helopeltis, Lygus, Dysdercus, Oxycarenus, Nezara, Aleurodes, Triatoma, Rhodnius, Psylla, Myzus, Megoura, Phylloxera, Adelyes, Niloparvata, Nephrotettix or Cimex spp.), Orthoptera (e.g. Locusta, Gryllus, Schistocerca or Acheta spp.), Dictyoptera (e.g. Blattella, Periplaneta or Blatta spp.), Hymenoptera (e.g. Athalia, Cephus, Atta Lasius, Solenopsis or Monomorium spp.), Isoptera (e.g. Odontotermes and Reticulitermes spp.), Siphonaptera (e.g. Ctenocephalides or Pulex spp.), Thysanura (e.g. Lepisma spp.), Dermaptera (e.g. Forficula spp.), Psocoptera (e.g. Peripsocus spp.) and Thysanoptera (e.g. Thrips tabaci),.

Acarine pests include ticks, e.g. members of the genera Boophilus,Ornithodorus, Rhipicephalus, Amblyomma, Hyalomma, Ixodes, Haemaphysalis, Dermacentor and Anocentor, and mites and manges such as Acarus, Tetranychus, Psoroptes, Notoednes, Sarcoptes, Psorergates, Chorioptes, Eutrombicula, Demodex, Panonychus, Bryobia and Eriophyes spp.

Nematodes which attack plants and trees of importance to agriculture, forestry, horticulture, either directly or by spreading bacterial, viral, mycoplasma or fungal diseases of the plants, include root-knot nematodes such as Meloidogyne spp. (e.g. M. incognita); cyst nematodes such as Globodera spp. (e.g. G. rostochiensis); Heterodera spp. (e.g. H. avenae); Radopholus spp. (e.g. R. similis); lesion nematodes such as Pratylenchus spp. (e.g. P. pratensis); Belonolaimus spp. (e.g. B. gracilis); Tylenchulus spp. (e.g. T. semipenetrans); Rotylenchulus spp. e.g.R. reniformis); Rotylenchus spp. (e.g. R. robustus); Helicotylenchus spp. (e.g. H. multicinctus);Hemicycliophora spp. (e.g. H. gracilis); Criconemoides spp. (e.g. C. similis); Trichodorus spp. (e.g. T primitivus); dagger nematodes such as Xiphinema spp. (e.g. X. diversicaudatum), Longidorus spp (e.g. L. elongatus); Hoplolaimus spp. (e.g. H. coronatus); Aphelenchoides spp. (e.g. A. ritzema-bosi, A. besseyi); stem and bulb eelworms such as Ditylenchus spp. (e.g. D. dipsaci).

Compounds of the invention may be combined with one or more other pesticidally active ingredients (for example pyrethroids, carbamates, lipid amides and organophosphates) and/or with attractants, repellents, bacteriocides, fungicides, anthelmintics and the like. Furthermore, the activity of compounds of the invention may be enhanced by the addition of a synergist or potentiator, for example: one of the oxidase inhibitor class of synergists, such as piperonyl butoxide or propyl 2-propynylphenyl-phosphonate; a second compound of the invention; or a pyrethroid pesticidal compound. When an oxidase inhibitor synergist is present in a formulation of the invention, the ratio of synergist to compound of Formula (I) will be in the range 500:1-1:25 eg about 100:1 to 10:1.

Stabilisers for preventing any chemical degradation which may occur with the compounds of the invention

include, for example, antioxidants (such as tocopherols, butylhydroxyanisole and butylhydroxytoluene) and scavengers (such as epichlorhydrin) and organic or inorganic bases e.g. trialkylamines such as triethylamine which can act as basic stabilisers and as scavengers.

The following Examples illustrate, in a non-limiting manner, preferred aspects of the invention. All temperatures are in degrees Celsius.

## EXPERIMENTAL

### General Synthetic Methods and Procedures:

Various compounds were synthesised and characterised in accordance with the following experimental procedures.

$^1H$ N.M.R. spectra were obtained on a Bruker AM-250 or WM-300 spectrometer in deuteriochloroform solutions with tetramethylsilane (TMS) as internal standard and are expressed as ppm from TMS, number of protons, number of peaks, coupling constant $J_{Hz}$.

Mass spectra were obtained on Finnigan 4500 or Hewlett Packard 5985B instruments. Gas-liquid chromatography (g.l.c.) was performed using a Pye Unicam GCD chromatograph fitted with a 3% OV210 column on Gas-Chrom Q and a flame-ionisation detector. Progress of reactions could also be conveniently monitored on plastic sheets (40 x 80 mm) precoated with 0.25 mm layers of silica gel with fluorescent indicator and developed in benzene. Temperatures are in degrees Celsius throughout.

### Section 1 Dithianes from 1,3-Dithiols

### Preparation of Intermediates in Dithiane Synthesis

#### 1. Dithiols

#### Method (a) 2-Isopropylpropan-1,3-dithiol

(i) Diethyl isopropylmalonate [Fluka, 15g, 0.074mol] in dry diethyl ether (20ml) was added to a suspension of lithium aluminium hydride (5.7 g, 0.15mol) in dry diethyl ether (300ml) under nitrogen at such a rate as to maintain reflux. The mixture was stirred for a further 1 hour, cooled and worked up by the addition of water (6ml), dilute (2N) sulphuric acid (6ml) then more water (6ml). The solid was filtered and washed with ether. The combined filtrates were dried ($MgSO_4$) and evaporated to give a colourless liquid (10.2g). This was taken on to the next stage without further purification.

(ii) Methanesulphonyl chloride (14ml, 0.175mol) was added to 2-isopropylpropan-1,3-diol (10g 0.074mol) in dry pyridine (100ml) at -10°C under nitrogen. The mixture was allowed to warm to room temperature and stirred for a further 4 hours. Water (100ml) and chloroform (200ml) were added, the aqueous layer separated and washed with chloroform (2 x 100ml) and the combined organic layers dried ($MgSO_4$) and evaporated to a pale yellow solid. The solid was triturated in dry ether to give an off-white crystalline solid (14.54g).

(iii)2-Isopropylpropan-1,3-diol dimethanesulphonate (25g, 0.091mol) was added to a mixture of freshly crushed sodium sulphide (24.4g [30%], 0.109mol) and sulphur (3.01g, 0.094mol) in dimethylformamide (250ml) and the mixture heated to reflux for 4 hours. After cooling, water/ice (500ml) was added followed by diethyl ether (300ml). The two layers were separated and the aqueous layer made acidic with concentrated hydrochloric acid. The aqueous layer was extracted with ether and the combined ethereal solutions washed with dilute (10%) hydrochloric acid, then water, dried ($MgSO_4$) and evaporated to give 4-isopropyl-1,2-dithiolane as an amber oil (12.01g).

(iv) 4-Isopropyl-1,2-dithiolane (12g) was taken up in dry diethyl ether (15ml) and added dropwise to a suspension of lithium aluminium hydride (6.1g, 0.081mol) in dry diethyl ether (250ml) under nitrogen with stirring. After stirring for a further 0.5 hour, water (10ml), dilute (2N) sulphuric acid (10ml) and more water (10ml) were added dropwise. The solid was filtered, washed with dry ether and the combined filtrates dried ($MgSO_4$) and evaporated to give 2-isopropylpropan-1,3-dithiol as a pale yellow liquid (8.25g).

$^1H$ nmr ($\delta CDCl_3$): 0.9 ($6H,d,CH_3$); 1.2 (2H,t,SH); 1.3-2.0 (2H,m,CH); 2.6 ($4H,dd,CH_2$).

2-Cyclopropylpropan-1,3-dithiol was similarly prepared from diethyl cyclopropylmalonate (Carney R.W.J., Wojtkunski J.; Org. Prep. and Procedures Int., 1873, 5(i), 25-29).

2-s-Butylpropan-1,3-dithiol was similarly prepared from diethyl s-malonate (Inui T., Kaneko T.; C.A. 5148f).

2-Isopropyl-2-methylpropan-1,3-dithiol was prepared using the above procedure.

Method (b) 2-Isopropylpropan-1,3-dithiol

( i) 2-Isopropylpropan-1,3-diol dimethanesulphonate (10g, 0.036mol) and sodium trithiocarbonate solution (12.4g, 0.08mol) were heated at reflux under nitrogen in dry dimethylformamide (100ml) for 4 hours. After cooling, dilute (2N) sulphuric acid (60ml) was added, followed by chloroform (200ml). The organic layer was separated, dried ($MgSO_4$) and evaporated down to give an amber liquid. This was taken up in hexane (200ml) washed with water (3x100ml), dried ($MgSO_4$) and evaporated to give 4-isopropyl-1,2-dithiolane as an orange oil (4.88g).

(ii) Reduction of 4-isopropyl-1,2-dithiolane using the methodology described in stage (iv) Method (a) gave 2-isopropylpropan-1,3-dithiol.

Method (c) 2-Cyclobutylpropane-1,3-dithiol

(i) Sodium (10.3g 0.44mol) was dissolved in dry ethanol (300ml) under a nitrogen atmosphere and to the cooled solution (0°C) was added diethyl malonate (71g, 0.44mol). After stirring for 15 minutes cyclobutyl bromide (60g, 0.44mol) was added and the solution was heated to reflux overnight. The cooled mixture was evaporated and the residue was partitioned between water and ether. The ether layer was separated, dried ($MgSO_4$) and evaporated. The residue was distilled to give diethylcyclobutyl malonate (40g, 42%) b.p. 74-75°C at 0.5mm Hg.

Nmr δ 4.1 (4H, q), 3.3 (1H,d), 2.8-2.9(1H,m), 1.7-2.1 (6H, m), 1.2 (6H,t).

ii) A solution of diethyl 2-cyclobutyl malonate (15g, 70mmol) in dry ether (10ml) was added to a stirred suspension of lithium alumnium hydride (3.8g, 100mmol) in dry ether (150ml) at 0°C under a nitrogen atmosphere. After the addition the solution was heated to gentle reflux for 1 hour, allowed to cool and an excess of saturated aqueous ammonium chloride was added. Stirring was continued overnight and the resulting mixture was filtered and the solids washed with ether. The filtrate was extracted with ether, the ether extracts were combined, dried ($MgSO_4$) and evaporated to leave 2-cyclobutylpropane-1,3-diol (8.65g, 95%) as a white solid m.p. 31-33°C.

Nmr δ 3.8-3.5 (4H, m) , 2.6(2H, s), 2.25-2.1 (1H, m), 2.05-1.65 (6H, m).

iii) To a stirred solution of 2-cyclobutylpropane-1,3-diol (8g, 61.5mmol) in dry chloroform (200ml) and pyridine(18ml, 0.22mol) at 0°C under nitrogen atmosphere was added methanesulphonyl chloride (15ml, 0.2mol). The mixture was stirred overnight at room temperature, poured into ice/water and extracted with chloroform. The chloroform extracts were combined, dried ($MgSO_4$) and evaporated to leave 2-cyclobutylpropane-1,3-diol dimethanesulphonate as a pale yellow gum (17.6g, 100%).

Nmr δ 4.2 (2H, d of d), 4.1 (2H, d of d), 3.0 (6H, s) 2.35 (1H, m), 2.15-1.70 (6H, m).

iv) To a stirred solution of benzylmercaptan (17g, 135mmol) in dry dimethylformamide (100ml) at 0°C under nitrogen atmosphere was added sodium hydride (4.0g of 80% oil dispersion ≡ 135mmol). The mixture was stirred for 30 minutes then 2-cyclobutylpropane-1,3-diol dimethanesulphonate (17.6g, 61.5mmol) was added and the mixture heated at 120°C overnight. The cooled solution was poured into ice/water, extracted with ether, the ether extracts were combined, dried ($MgSO_4$) and evaporated to leave 2-cyclobutylpropane-1,3-dibenzylthioether as a yellow-orange oil (crude yield 24.67g).

Nmr δ 7.35-7.2 (10H, m), 3.65 (4H, s), 2.55 (2H, d of d), 2.45 (2H, d of d), 2.25 (1H, m), 1.95-1.50 (6H, m).

v) To liquid ammonia (1l) was added a solution of 2-cyclobutylpropane-1,3-dibenzylthioether (24.67g, 61.5mmol) in dry ether (50ml). The resulting mixture was stirred and sodium pellets (9.2g, 0.4mol) were added. After stirring for 3 hours, ammonium chloride (22g, 0.41mol) was added and the ammonia was allowed to evaporate overnight. The resulting residue was washed with ether, filtered and the filtrate was evaporated to leave 2-cyclobutylpropane-1,3-dithiol as a pale yellow oil (crude yield 10.43g).

Nmr δ 2.75 (2H, m), 2.60 (2H, m), 2.40 (1H, m), 2.1 - 1.65 (7H, m), 1.20 (2H, t).

Method (d) 2-(1-Methylcyclopropyl)propane-1,3-dithiol

Using the above procedure this dithiol (6.6g, 74%) was prepared as a yellow oil from diethyl 2-(1-methylcyclopropyl)malonate.

Nmr δ 2.7 (4H, m), 1.45 (2H, t), 0.9 (3H, s), 0.9 (1H, m), 0.45 (2H, m), 0.35 (2H, m).

The malonate was prepared as follows:-

1-Methylcyclopropanecarbonyl chloride (58.77g, 0.5mol) was added dropwise to a stirred solution of ethyl diazoacetate (114g, 1mol) in dry ether (500ml) at 0°C under a nitrogen atmosphere. The mixture was allowed to warm to room temperature then was stirred for 1 day, heated to gentle reflux for 2 days, then left to stand at room temperature for 5 days. Evaporation of the mixture followed by distillation up to 40°C at 1mm Hg left

ethyl 2-diazo-3-(1-methylcyclopropyl)-3-oxopropanoate (41g, 42%) as a yellow liquid residue.
Nmr δ 4.25 (2H, q), 1.35(3H, s), 1.30 (3H, t), 1.05 (2H, m), 0.65 (2H, m).

A mixture of the above diazoester (41g, 0.21mol) and silver oxide (400mg) in anhydrous toluene (50ml) was heated to reflux under an atmosphere of carbon dioxide for 4 hours. The cooled solution was evaporated and the residue was distilled into a flask containing dry ethanol (60ml) cooled to -70°C. The ketene distilled slowly b.p. 60-85°C at 1mm Hg. The ethanol solution was allowed to warm to room temperature and was then evaporated to leave diethyl 2-(2-methylcyclopropyl)malonate (18g, 40%).
Nmr δ 4.15 (4H, q), 2.75 (1H, s), 1.25 (6H, t), 1.2 (3H, s), 0.55 (2H, m), 0.45 (2H, m).

Method (e) 2-(1-Trifluoromethylethyl)propane-1,3-dithiol

Using the procedure of Method (a) this dithiol was prepared from diethyl (1-trifluoromethylethyl)malonate.

The malonate was prepared as follows :-

Diethyl trifluoroisopropylidenemalonate (7g) (Lehnert W., Tetrahedron, 1973,29 635) dissolved in ethyl acetate (100ml) was hydrogenated at atmospheric pressure using 5% palladim on carbon as the catalyst. Filtration through celite and evaporation of the filtrate yielded diethyl (1-trifluoromethylethyl)malonate as a colourless liquid (7g).
Nmr δ 1.2(3H,d); 1.3(6H,t); 3.1(1H,m); 3.6(1H,d); 4.2(4H,q).

Method (f) 2-(1-Methyl-1-trifluoromethylethyl)propan-1,3-diol

Using the procedure of Method (a) this dithiol was prepared from diethyl (1-methyl-1-trifluoromethylethyl)malonate.

The malonate was prepared as follows :-

Methyl iodide (8.95g) in diethyl ether (10ml) was added dropwise to a suspension of magnesium (1.43g) in dry diethyl ether (30ml). The mixture was cooled to -5° and copper (I) chloride (0.2g) was added. A solution of diethyl trifluoroisopropylidenemalonate (10g) in diethyl ether (20ml) was added at such a rate as to maintain the temperature below 0°. After stirring for 1 hour the mixture was poured onto ice/water and diethyl ether (100ml) and 2N sulphuric acid were added. The solutions were separated and the organic phase dried ($MgSO_4$) and evaporated to give diethyl (1-methyl-1-trifluoromethylethyl) malonate as an amber oil (9.7g).
Nmr δ 1.3(6H,t); 1.5(6H,s); 3.7(1H,s); 4.2(4H,q).

2. Aldehydes and Ketones Used in Dithiane Synthesis

Process A

4-(3-Hydroxyprop-1-ynyl )acetophenone

To 4-bromoacetophenone (3g) in triethylamine (60ml) was added propargyl alcohol (1ml); bis-triphenylphosphinepalladium dichloride (165.6mg) and copper(I)iodide (66mg). The mixture was stirred under nitrogen overnight. Ether was added and the mixture filtered. The filtrate was washed with water, dried over anhydrous magnesium sulphate and evaporated. The crude material was purified by column chromatography on silica eluting with ether:hexane.

4-(3-Hydroxyprop-1-ynyl)benzaldehyde, 4-(2-trimethylsilylethynyl)benzaldehyde and 4-(2-trimethylsilylethynyl)acetophenone were prepared in an analogous manner.

Process B

4-(3-Acetoxyprop-1-ynyl)benzaldehyde

To 4-(3-hydroxyprop-1-ynyl)benzaldehyde (500mg.) in dry benzene (20ml) was added acetic anhydride (326mg) and anhydrous sodium acetate (112mg.). The mixture was heated at reflux for 4 hours. After cooling, water (50ml) was added followed by ether (50ml.). The organic layer was separated and washed with sodium carbonate solution (2x50ml), washed with water, dried over anhydrous magnesium sulphate and evaporated in vacuo. The product was isolated by column chromatography on silica eluting with ether:hexane.

4-(3-Benzoyloxyprop-1-ynyl)benzaldehyde was prepared in an analogous manner.

Process C

4-Ethynylcyclohexanecarboxaldehyde

i) Di-isopropylamine (44.7ml) was dissolved in dry tetrahydrofuran (400ml) and cooled to -78°C under nitrogen with mechanical stirring. A solution of n-butyllithium in hexane (1.6M, 197ml) was added. After stirring at -78°C for 10 minutes a solution of dimethylcyclohexane-1,4 -dicarboxylate ((52.6g) Lancaster) in tetrahydrofuran (200ml) was added. After stirring for a further 30 minutes at -78°C a solution of acetyl chloride (22.5ml) in tetrahydrofuran (200ml) was added. The reaction mixture was allowed to warm to room temperature over a period of 3 hours. Water was then added and the mixture extracted with ether. The ethereal extracts were washed with water, saturated sodium bicarbonate solution, dilute hydrochloric acid and brine, and were then dried over anhydrous magnesium sulphate. Evaporation under reduced pressure gave a colourless oil which was slowly distilled to yield dimethyl 1-acetylcyclohexane-1,4-dicarboxylate (23.3g, b.p. 114-120°/0.4mmHg).

ii) Dimethyl 1-acetylcyclohexane-1,4-dicarboxylate (23.3g) was added to a solution of concentrated hydrochloric acid (253ml) in methanol (126ml). After refluxing for 10 hours the reaction mixture was poured into water and then extracted with dichloromethane. The organic phase was then washed with saturated sodium bicarbonate solution and brine. After drying over anhydrous magnesium sulphate the solvent was removed under reduced pressure to give methyl 4-acetylcyclohexanecarboxylate as a colourless oil. This was purified by distillation (b.p. 138-145°/14mmHg).

iii) Methyl 4-acetylcyclohexanecarboxylate (1.0g) in dry pyridine (0.7ml) was added to a stirred mixture of phosphorous pentachloride (2.45g) in dry pyridine (1.4ml). After stirring under reflux for 8 hours the reaction mixture was quenched by pouring into water. The mixture was then extracted with ether and the organic extracts washed with dilute hydrochloric acid, saturated sodium bicarbonate solution and brine. After drying over anhydrous magnesium sulphate, the solvent was removed under reduced pressure to give methyl 4-(1-chloroethenyl) cyclohexanecarboxylate as a pale yellow oil.

iv) Lithium aluminium hydride (283mg) was added to dry ether at 0° under a stream of nitrogen. After addition of methyl 4-(1-chloroethenyl) cyclohexanecarboxylate (1.0g) the reaction mixture was allowed to warm to 25° over a period of 2 hours. Sodium hydroxide solution (2.5ml., 10%) was added cautiously. The ethereal solution was then decanted from the mixture, dried and evaporated to give 4-(1-chloroethenyl) cyclohexylmethanol.

v) n-Butyllithium (12ml., 1.6M) was added at 0° under nitrogen to a stirred solution of 4-(1-chloroethenyl)cyclohexylmethanol (0.84g) in dry tetrahydrofuran (15ml). The reaction mixture was allowed to warm to room temperature and was stirred at 25° for 4 hours. Ice/water (100ml) was then added and the reaction mixture extracted with diethyl ether. After washing the organic extracts with brine and drying over anhydrous magnesium sulphate, the solvent was removed under reduced pressure. 4-Ethynylcyclohexylmethanol was purified by column chromatography on silica (eluted with ether:hexane; 2:3).

vi) Oxalyl chloride (354 l) was dissolved in dry dichloromethane (3ml) at -70° under nitrogen. Dimethyl sulphoxide (650 l) in dichloromethane (3ml) was then added. After stirring for 5 minutes a solution of 4-ethynylcyclohexylmethanol (0.5g) in dichloromethane (5ml) was added dropwise over 5 minutes. The reaction mixture was stirred for 30 minutes at -70° before triethylamine (2.5ml) was added. After warming to 25° over 3 hours, water was added and the organic phase separated washed with hydrochloric acid, saturated sodium bicarbonate solution and brine, and dried. Evaporation gave 4-ethynylcyclohexanecarboxaldehyde as a colourless oil.

Using the methodology in stage (vi) of Process C, hept-6-ynal was prepared from hept-6-yn-1-ol (C. Crisan Chem. Abs. 51:5061b).

The preparation of hept-2-en-6-ynal and 1,1,1-trichloropentan-4-one is described in EP 029 4 229.

3. Methods of Preparation of Dithianes from 1,3-Dithiols and Dithianes

Method 1

5-Isopropyl-2-(4-bromophenyl)-1,3-dithiane

A mixture of 4-bromobenzaldehyde (0.66g.,3.6mmol), 2-isopropylpropane-1,3-dithiol(0.53g,3.5mmol) and p-toluenesulphonic acid (50mg) in benzene (100ml) was refluxed in a Dean and Stark apparatus for 6 hours. After cooling the mixture was poured into water and the aqueous mixture was extracted with diethyl ether. The organic extracts were washed with water, dried over anhydrous magnesium sulphate and evaporated in vacuo.

The residue was recrystallised from hexane and 2-(4-bromophenyl)-5-isopropyl-1,3-dithiane was obtained as a colourless crystalline solid (0.88g).

SECTION 2 Dithianes from Dithiane Precursors

Method 2

5-Isopropyl-2-(4-ethynylphenyl)-1,3-dithiane

A solution of 5-isopropyl-2-(2-trimethylsilylethynylphenyl)-1,3-dithiane (0.55g) in dry tetrahydrofuran (20ml) was stirred under nitrogen at room temperature while a solution of tetrabutylammonium fluoride in tetrahydrofuran (1M,2ml) was added. After stirring overnight the solvent was removed and water and ether added. The ethereal solution was separated, dried over magnesium sulphate and evaporated. Chromatography on silica eluting with ether:hexane and crystallisation from hexane gave 5-isopropyl-2-(4-ethynylphenyl)-1, 3-dithiane (157mg), m.p.= 134°.

Method 3

trans 5-Isopropyl-2-(4-bromophenyl)-1,3-dithiane 1,3-dioxide

m-Chloroperbenzoic acid (0.58g) was added to a stirred suspension of sodium acetate (1.2g) and trans-5-isopropyl-2-(4-bromophenyl)-1,3-dithiane (0.5g) in dry acetonitrile (50ml). After stirring at room temperature for 2 hours a further quantity of m-chloroperbenzoic acid (0.5g) was added. Stirring was continued for 2 hours and the mixture filtered and the residue washed with diethyl ether. The combined filtrates were evaporated, extracted with chloroform and the organic solution washed with water, dried over magnesium sulphate and evaporated. The residue was chromatographed on silica eluting with ethyl acetate to give trans-5-isopropyl-2-(4-bromophenyl)-1,3-dithiane 1,3-dioxide (37mg).

Further compounds were prepared from appropriate starting materials; using similar methodology these compounds and their physical and chemical characteristics are listed in Tables 1, 2 and 3.

**TABLE 1**

| Compound Number | $R^{2a}$ | $R^{2b}$ | $R^{5a}$ | $R^{5b}$ | trans/cis Isomer Ratio | m | n |
|---|---|---|---|---|---|---|---|
| 1 | 4-Bromophenyl | H | isopropyl | H | 95:5 | 0 | 0 |
| 2 | 4-Bromophenyl | H | isopropyl | H | 1:2 | 0 | 0 |
| 3 | 4-Trimethylsilylethynylphenyl | H | isopropyl | H | 9:1 | 0 | 0 |
| 4 | 4-Ethynylphenyl | H | isopropyl | H | trans | 0 | 0 |
| 5 | 4-Ethynylphenyl | H | isopropyl | H | 1:2 | 0 | 0 |
| 6 | 4-Ethynylphenyl | Me | isopropyl | H | 5:7 | 0 | 0 |
| 7 | 4-(3-Acetoxyprop-1-ynyl)phenyl | H | isopropyl | H | trans | 0 | 0 |
| 8 | 4-(3-Acetoxyprop-1-ynyl)phenyl | H | isopropyl | H | 2:3 | 0 | 0 |
| 9 | 4-(3-Benzoyloxyprop-1-ynyl)phenyl | H | isopropyl | H | trans | 0 | 0 |

| Compound Number | $R^{2a}$ | $R^{2b}$ | $R^{5a}$ | $R^{5b}$ | trans/cis Isomer Ratio | m | n |
|---|---|---|---|---|---|---|---|
| 10 | 4-(3-Benzoyloxyprop-1-ynyl)phenyl | H | isopropyl | H | 7:3 | 0 | 0 |
| 11 | 4-(e)-Ethynylcyclohexyl | H | isopropyl | H | 4:1 | 0 | 0 |
| 12 | 4-(e)-Ethynylcyclohexyl | H | isopropyl | H | 1:1 | 0 | 0 |
| 13 | Hex-5-ynyl | H | isopropyl | H | 9:1 | 0 | 0 |
| 14 | 4-(e)-Ethynylcyclohexyl | H | isopropyl | H | isomer | 1 | 0 |
| 15 | 4-Bromophenyl | H | isopropyl | H | trans | 1 | 1 |
| 16 | 4-Ethynylphenyl | H | isopropyl | H | trans | 1 | 0 |
| 17 | 4-Ethynylphenyl | H | isopropyl | H | cis | 0 | 0 |
| 18 | 4-(e)-Ethynylcyclohexyl | H | isopropyl | H | 2:1 | 0 | 0 |
| 19 | 4-(e)-Ethynylcyclohexyl | H | isopropyl | H | trans | 1 | 0 |

| Compound Number | $R^{2a}$ | $R^{2b}$ | $R^{5a}$ | $R^{5b}$ | trans/cis Isomer Ratio | m | n |
|---|---|---|---|---|---|---|---|
| 20 | (E)-Hex-1-en-5-ynyl | H | isopropyl | H | trans | 0 | 0 |
| 21 | 3,3,3-Trichloropropyl | Me | isopropyl | H | 5:1 | 0 | 0 |
| 22 | 4-Trimethylsilylethynylphenyl | H | 1-trifluoro-methylethyl | H | 1:1 | 0 | 0 |
| 23 | 4-Ethynylphenyl | H | 1-trifluoro-methylethyl | H | 1:4 | 0 | 0 |
| 24 | 4-Ethynylphenyl | H | 1-trifluoro-methylethyl | H | trans | 0 | 0 |
| 25 | 4(e)-Ethynylcyclohexyl | H | 1-trifluoro-methylethyl | H | trans | 0 | 0 |
| 26 | 4-Ethynylphenyl | H | 1-trifluoro-methylethyl | H | 1:1 | 0 | 0 |

| Compound Number | $R^{2a}$ | $R^{2b}$ | $R^{5a}$ | $R^{5b}$ | trans/cis Isomer Ratio | m | n |
|---|---|---|---|---|---|---|---|
| 27 | 4-Trimethylsilylethynylphenyl | H | 1,1-dimethyl-2,2,2-trifluoroethyl | H | 3:2 | 0 | 0 |
| 28 | 4-Ethynylphenyl | H | 1,1-dimethyl-2,2,2-trifluoroethyl | H | trans | 0 | 0 |
| 29 | 4-Ethynylphenyl | H | 1,1-dimethyl-2,2,2-trifluoroethyl | H | 1:1 | 0 | 0 |
| 30 | 4-Ethynylphenyl | H | 1,1-dimethyl-2,2,2-trifluoroethyl | H | trans | 1 | 0 |
| 31 | 4-Ethynylcyclohexyl | H | cyclopropyl | H | Isomer mixture | 0 | 0 |
| 32 | 4-Trimethylsilylethynylphenyl | H | H | cyclopropyl | cis | 0 | 0 |

| Compound Number | $R^{2a}$ | $R^{2b}$ | $R^{5a}$ | $R^{5b}$ | Isomer Ratio | m | n |
|---|---|---|---|---|---|---|---|
| 33 | 4-Ethynylphenyl | H | cyclopropyl | H | trans | 0 | 0 |
| 34 | 4-Ethynylphenyl | H | cyclobutyl | H | trans | 0 | 0 |
| 35 | 4-Ethynylphenyl | H | 1-methylcyclo-propyl | H | trans | 0 | 0 |
| 36 | 4-Bromophenyl | H | cyclobutyl | H | trans | 0 | 0 |
| 37 | 4-(e)-Ethynylcyclohexyl | H | isopropyl | H | cis | 0 | 0 |
| 38 | 4-(e)-Ethynylcyclohexyl | H | isopropyl | H | trans | 0 | 0 |
| 39 | 4-Ethynylphenyl | H | isopropyl | methyl | 3:2 | 0 | 0 |
| 40 | 4-Ethynylphenyl | H | s-butyl | H | 9:1 | 0 | 0 |
| 41 | 4-Trimethylsilylethynylphenyl | H | s-butyl | H | trans | 0 | 0 |

TABLE 2

Nuclear Magnetic Resonance Spectra:- 1H,CDC$1_3$, and expressed as p.p.m. downfield from TMS (number of protons, multiplicity, assignment).

1. trans 0.98(6H,d,$CMe_2$); 1.55-1.8(2H,m,5-H+CH); 2.85 (4H,m,4-and 6-$H_2$); 5.05(1H,5,2.H); 7.3 (2H,d,ArO; 7.48(2H,d,Ar).

cis 0.98(6H,d,$CMe_2$); 1.55-1.80 (2H,m,5-H+CH); 2.8-3.1 (4H,m,4-and 6-$H_2$); 7.3(2H,d,Ar); 7.48(2H,d,Ar).

3. trans 0.25 (9H,s,$SiMe_3$); 0.95 (6H,d,$CMe_2$); 1.5-1.85(2H,m,5-H+CH); 2.85 (4H,m,4- and 6-$H_2$); 5.1 (1H,s,2-H); 7.4 (4H,m,Ar).

cis 0.25(9H,s,$SiMe_3$); 0.95 (6H,d,$CMe_2$); 1.55-1.85 (2H,m, 5-H + CH); 2.8-3.1 (4H,m,4- and 6-$H_2$); 5.05(1H,s,2-H); 7.4(4H,m,Ar).

4. trans 0.95 (6H,d,$CMe_2$); 1.5-1.85(2H,m,5-H+CH); 2.85(4H,m,4- and 6-$H_2$); 3.12(1H,s,-C≡H); 5.14(1H,s,2-H); 7.5(4H,m,Ar).

5. trans 0.95(6H,d,$CMe_2$); 1.5-1.85(2H,m, 5-H + CH); 2.85 (4H,m, 4- and 6-$H_2$); 3.12(1H,s,≡CH); 5.14(1H,s,2-H); 7.5(4H,m,Ar).

cis 0.95 (6H,d,$CMe_2$); 1.55-1.85(2H,m, 5-H + CH); 2.85-3.15(4H,m,4- and 6-$H_2$); 3.12(1H,s,≡CH); 5.05(1H,s,2-H); 7.4(4H,m,Ar).

6. trans 0.95(6H,d,$CMe_2$); 1.4-1.8(2H,m,5-H+CH); 1.7(3H,s,2-Me); 2.3-2.9(4H,m, 4- and 6-$H_2$): 3.1(1H,s,≡CH); 7.4(4H,m,Ar).

cis 0.85(6H,d,$CMe_2$); 1.4-1.8(2H,m, 5-H + CH); 1.9(3H,s,2-Me); 2.3-2.9 (4H,m,4- and 6-$H_2$); 3.1 (1H,s,≡CH); 7.4(4H,m,Ar).

7. trans 0.98(6H,d,$CMe_2$); 1.6-1.85(2H,m,5-H+CH); 2.15(3H,s,COMe); 2.85(4H,m, 4- and 6-$H_2$); 4.9(2H,s,$OCH_2$); 5.2(1H,s,2-H); 7.4 (4H,m,Ar).

8. trans 0.98(6H,d,$CMe_2$); 1.6-1.85(2H,m, 5-H + CH); 2.15(3H.s.COMe); 2.85(4H,m,4- and 6-$H_2$); 4.9(2H,s,$OCH_2$); 5.12(1H,s,2-H); 7.4 (4H,m,Ar).

cis 0.98r6h,d,$CMe_2$); 1.55-1.85(2H,m,5-H + CH); 2.15(3H,s,COMe); 2.9-3.1(4H,m, 4- and 6-$H_2$); 4.9(2H,s,$OCH_2$); 5.1(1H,s, 2-H); 7.4(4H, m,Ar).

9. trans 0.95 (6H,d,$CMe_2$); 1.6-1.85(2H,m,5-H + CH); 2.85(4H,d, 4- and 6-$H_2$); 5.1(1H,s,2-H); 5.15(2H,s,$OCH_2$); 7.4-7.6 (7H,m,Ar); 8.1(2H,d,Ar)

10. trans 0.95 (6H,d, $CMe_2$); 1.6-1.85(2H,m,5-H + CH); 2.85(4H,m 4- and 6-$H_2$); 5.1(1H,s,2-H); 5.15(2H,s,$OCH_2$); 7.4-7.6(7H,m,Ar); 8.1(2H,d,Ar).

cis 0.95 (6H,d,$CMe_2$); 1.55-1.6 (2H,m,5-H + CH); 2.35-3.12(4H,m, 4- and 6-$H_2$); 5.1(1H,s, 2-H); 5.15(2H,s,$OCH_2$);7.4-7.6(7H,m,Ar); 8.1(2H,d,Ar).

11. trans 0.9 (6H,d,$CMe_2$); 1.15-1.45(4H,m,$CH_2$); 1.5-1.75(4H,m,CH); 1.9-2.2(5H,m,CH + $CH_2$); 2.6-2.9(4H,m,4- and 6-$H_2$); 3.98(1H,d,2-H).

cis 0.92(6H,d,$CMe_2$); 1.15-1.45(4H,m,$CH_2$); 1.5-1.8(4H,m,CH); 1.9-2.2(5H,m,CH + $CH_2$); 2.6-2.9(4H,m, 4- and 6-$H_2$); 3.88(1H,d,2-H).

13. trans 0.94(6H,d,$CMe_2$); 1.5-1.75(8H,m, 5-H, + $CH_2$); 1.95(1H,t,=CH); 2.2(2H,m,$CH_2$); 2.6-2.8(4H,m, 4- and 6-$H_2$); 4.0(1H,t, 2-H).

cis 0.94(6H,d,$CMe_2$); 1.5-1.8(8H, 5-H + $CH_2$), 1.95(1H,t,=CH); 2.2 (2H,m,$CH_2$); 2.6-2.9(4H,m, 4- and 6-$H_2$); 3.88(1H,s,2-H).

14. 0.88-0.95 1.1 (6H,d's. $CMe_2$); 1.2-1.75(7H,m,CH's + $CH_2$'s); 1.9-2.25 (6H,m, CH's + $CH_2$'s); 2.35-3.85(4H,m, 4- and 6-$H_2$); 3.45-3.55(1H, d's,2-H).

15. trans 1.05(6H,d,$CMe_2$); 1.9-2.1(2H,m, 5-H + CH); 2.9(2H,m,$CH_2$); 3.55 (2H,m,$CH_2$); 4.62(1H,s,2-H); 7.45(2H,d,Ar); 7.65(2H,d,Ar).

16. 0.95(6H,d,$CMe_2$); 1.65(1H,m,C$\underline{H}Me_2$); 2.3(1H,m,C5-H); 2.6-3.6(5H,m,); 4.5(1H,s,C2-4); 7.4(4H,m,Ar).

17. 0.95(6H,d,$CMe_2$); 1.4(1H,m,C5-H); 2.65(m,1H,C$\underline{H}Me_2$); 2.95(2H,dd,C4-e); 3.05(1H,s,≡C$\underline{H}$); 3.15(2H,dd,C4-a); 5.15(1H,s,C2-H); 7.5(4H,m,Ar).

18. 0.95(6H,d,$CMe_2$); 1.1-2.3(12H,m); 2.5-2.9(5H,m); 3.9 and 4.0(1H,d,C2-H).

19. 0.95(6H,d,$CMe_2$); 1.0-2.3(12H,m); 2.3-3.4(5H,m); 3.5(1H,d,C2-H).

20. 0.95(6H,d,$CMe_2$); 1.5-2.4(6H,m); 2.2(4H,m); 4.6(1H,d,C2-H);5.8(2H,m,=C$\underline{H}$).

21. 0.95(6H,m,$CMe_2$); 1.4(1H,m,C5-H); 1.5 and 1.7(3H,s,C2-Me);1.9(1H,m,C$\underline{H}Me_2$); 2.3-3.1(8H,m).

22. 0.2(9H,s,$SiMe_3$); 1.2(3H,m,Me); 2.4(1H,m); 1.7-3.2(4H,m); 5.1(1H,s,C2-H); 7.4(4H, Ar).

23. 1.2(3H,m,Me); 2.1(1H,m); 2.9-3.5(5H,m); 5.0 and 5.1(1H,s,C2-H); 7.4(4H,m,Ar).

24. 1.2(3H,m,Me); 2.4(1H,m); 2.7-3.2(5H,m); 5.1(1H,s,C2-H); 7.4(4H,m,Ar).

25. 1.1(3H,m,Me); 1.2-2.3(12H,m); 2.8(5H,m); 4.0(1H,d,C2-H);

26. 1.2(3H,m,Me); 2.1(1H,m); 2.9-3.5(5H,m); 5.0 and 5.1(1H,s,C2-H); 7.4(4H,m,Ar).

27. 0.2(9H,s,Si$\underline{Me}_3$); 1.1(6H,s,C$\underline{Me}_2$); 2.2(1H,m); 2.7(4H,m); 4.8(1H,s,C2-H); 7.5(4H,Ar).

28. 1.2(6H,s,$C\underline{Me_2}$); 2.2(1H,m); 2.9(4H,m); 3.0(1H,s,≡CH); 5.1(1H,s,C2-H); 7.4(5H,Ar).
29. 1.1 and 1.2(6H,s,$C\underline{Me_2}$); 2.2(1H,m); 2.8(4H,m); 3.0(1H,s,≡C$\underline{H}$); 4.8 and 5.1(1H,s,C2-H); 7.5(5H,Ar).
30. 1.2(6H,m,$CMe_2$); 2.5-3.7(4H,m); 3.1(1H,s,≡CH); 4.5(1H,s,C2-H); 7.4(4H,m,Ar).
31. 0.2-1.0(5H,m,cyclopropyl-H); 1.2-2.3(11H,m); 2.6-3.1(5H,m); 3.8 and 4.0(1H,m).
32. 0.15(2H,m,cyclopropyl); 0.2(9H,s,$SiMe_3$); 0.6(2H,m,cyclopropyl); 1.0(1H,m,cyclopropyl); 1.6(1H,m,C5-H); 2.7-3.2(4H,m); 5.0(1H,s,C2-H); 7.4(4H,m,Ar).
33. 0.2, 0.5 and 1.0(5H,m,cyclopropyl); 2.8(4H,m); 3.0(1H,s,≡CH); 5.1(s,C2-H); 7.4(4H,m,Ar).
34. 1.7-2.1(8H,m); 2.55(2H,dd); 2.8(2H,dd); 3.05(1H,s); 5.05(1H,s); 7.4(4H,m,Ar).
35. 0.35(4H,m); 0.95(3H,s); 1.2(1H,tt); 2.85(2H,dd): 3.05(1H,s); 3.05(2H,dd); 5.1(1H,s); 7.4(4H,m,Ar).
36. 1.7-2.1(8H,m); 2.55(2H,dd); 2.8(2H,dd); 5.0(1H,s); 7.4(4H,m,Ar).
37. 0.95(6H,d,$C\underline{Me_2}$); 1.1-1.8(6H,m); 2.0-2.4(6H,m); 2.1(1H,d,≡CH); 2.9(4H,m); 3.9(1H,d,C2-H).
38. 0.9(6H,d,$C\underline{Me_2}$); 1.2-1.8(6H,m); 1.9-2.3(6H,m); 2.1(1H,d,≡CH); 2.6-2.9(4H,m); 4.0(1H,d,C2-H).
39. 0.9(6H,m,$C\underline{Me_2}$); 1.6(1H,m); 2.5-3.0(4H,m); 3.1(1H,s,≡C$\underline{H}$); 5.0 and 5.1(1H,s,C2-H); 7.5(4H,Ar).
40. 0.9(6H,m,2xMe); 1.0-2.0(4H,m); 2.8(4H,m); 3.1(1H,s,≡CH); 5.1(1H,s,C2-H); 7.4(4H,m,Ar).
41. 0.2(9H,s,$SiMe_3$); 0.9(6H,m,2xMe); 1.1-2.0(4H,m); 2.8(4H,m); 5.1(1H,s,C2-H); 7.3(4H,m,Ar).

**TABLE 3** DITHIANES - Further Characterising Data

| Compound Number | Method of Preparation | Mass Spectrum Chemical Ionisation M+1 | M.p. $^{o}$C or $N_D$. | Description |
|---|---|---|---|---|
| 1 | 1 | 317 | 139 | Colourless Crystalline Solid |
| 2 | 1 | 317 | 115-6 | Colourless Crystalline Solid |
| 3 | 1 | 335 | 131 | Colourless Crystalline Solid |
| 4 | 2 | 263 | 134 | Colourless Crystalline Solid |
| 5 | 2 | 263 | 85 | Pale Yellow Crystalline Solid |
| 6 | 2 | 277 | 1.590 | Yellow Oil |
| 7 | 1 | 335 | 96 | Pale Yellow Crystalline Solid |
| 8 | 1 | 335 | 89 | Pale Yellow Crystalline Solid |
| 9 | 1 | 397 | 131 | Colourless crystals |
| 10 | 1 | 397 | 105 | Colourless crystals |
| 11 | 1 | 269 | 72 | Colourless crystals |
| 12 | 1 | 269 | | Colourless crystals |
| 13 | 1 | 243 | 32 | Colourless crystals |
| 14 | 3 | 285 | 142 | Colourless crystals |
| 15 | 3 | 349 | 188 | Colourless Crystalline Solid |
| 16 | 3 | 279 | 188-9 | Pale Yellow Solid |

| Compound Number | Method of Preparation | Mass Spectrum Chemical Ionisation M+1 | M.p.° C or $N_D$. | Description |
|---|---|---|---|---|
| 17 | 2 | 263 | 104 | White Solid |
| 18 | 1 | 269 | 55 | White Solid |
| 19 | 3 | 285 | 163 | Pale Yellow Solid |
| 20 | 1 | 241 | - | Yellow Oil |
| 21 | 1 | - | - | Brown Oily Solid |
| 22 | 1 | 389 | - | Pale Yellow Solid |
| 23 | 2 | 317 | 94 | Yellow Solid |
| 24 | 2 | 317 | 109 | Off White Solid |
| 25 | 1 | 323 | 62 | Pale Yellow Solid |
| 26 | 2 | 317 | 100 | White Solid |
| 27 | 1 | 403 | 147 | White Solid |
| 28 | 1 | 331 | 150 | Pale Yellow Solid |
| 29 | 2 | 331 | 104 | Pale Yellow Solid |
| 30 | 3 | 347 | 166 | Colourless Solid |
| 31 | 1 | 267 | 1.563 | Pale Yellow Oil |
| 32 | 1 | 333 | 117-8 | Colourless Solid |
| 33 | 2 | 261 | 143 | Colourless Crystals |

| Compound Number | Method of Preparation | Mass Spectrum Chemical Ionisation M+1 | M.p.° C or $N_D$. | Description |
|---|---|---|---|---|
| 34 | 2 | 275 | 149-151 | White Solid |
| 35 | 2 | 275 | 149-152 | White Crystals |
| 36 | 1 | 331 | 148-9 | White Crystals |
| 37 | 1 | 269 | 90 | White Solid |
| 38 | 1 | 269 | 88 | White Solid |
| 39 | 2 | 277 | 83 | Pale Yellow Solid |
| 40 | 2 | 277 | - | Clear Oil |
| 41 | 1 | 349 | 110-1 | White Solid |

BIOLOGICAL ACTIVITIES

The activity of the compounds of the invention were tested by dissolving the compounds in acetone (5%) and then diluting in water: ;Symperonic' (94.5% : 0.5%) to give a water emulsion. The solution was then used to treat the following insects.

Musca domestica:

The initial activity of the compound of invention against female Musca domestica (WRL strain) was assessed by the spraying of compound solution over a mesh covered cylinder containing 20 files. Mortality was assessed after 24 and 48 hours and included knockdown data.

The following compounds were active at less than 1000 ppm:
1, 2, 6, 7, 8, 9, 20.

The following compounds were active at less than 200 ppm:
4, 5, 11, 14, 12, 13, 17, 19, 21, 22, 23, 24, 25, 27, 28, 29, 34, 35, 40.

Plutella xylostella:

The activity of compounds of the invention against 2nd instar Plutella xylostella (WRL strain) larvae was demonstrated by the spraying of compound solution on a leaf disc infested with the larvae. Mortality was assessed after 48 hours.

The following compounds were active at less than 1000 ppm:
3, 4, 6, 13, 17, 21, 25, 40.

The following compounds were active at less than 500 ppm:
5, 11, 14, 12, 19, 20, 23, 24, 29, 31, 35, 38.

Myzus persicae:

The activity of compounds of the invention against adult Myzus persicae (WRL strain) was demonstrated by the spraying of compound solution on a leaf disc infested with Aphids. Mortality was assessed after 48 hours.

The following compounds were active at less than 1000 ppm:
2, 6, 8, 27, 34.

The following compounds were active at less than 500 ppm:
20, 24, 25, 28.

The following compounds were active at less than 200 ppm :
4, 5, 7, 16, 17, 19, 21, 23, 29, 31, 35, 38.

Spodoptera littoralis

Leaf discs were sprayed with the solution containing the compound. Ten 1st instar Spodoptera littoralis (WRL strain) larvae were then added to the leaf discs. Mortality was assessed after 72 hours.

The following compounds were active at less than 1000 ppm:
20, 31, 38.

The following compounds were active at less than 500 ppm:
25

Tetranychus urticae:

Solutions of the compounds of invention were sprayed on leaf discs infested with populations of all the life stages of Tetranychus urticae (WRL strain). Mortality was assessed after 48 hours.

The following compounds were active at less than 1000 ppm:
9, 19, 20, 22, 23, 24.

Diabrotica undecimpunctata

Filter paper was sprayed with the solution containing the compound. Ten 2nd instar larvae were then added to the filter paper together with a cube of artificial diet. Activity was assessed after 48 hours.

The following compounds were active at less than 1000 ppm:

2, 6, 12, 14, 16, 17, 20, 21, 22, 27, 29, 35, 40.

The following compounds were active at less than 200 ppm:

4, 5, 11, 19, 23, 24, 25, 28, 31.

Sitophilus granarius:

20 adult sitophilus were added to 10g wheat which had been previously treated with 2ml of this solution containing the compound. Mortality is assed after 6 days at 25°C.

The following compounds were active at less than 1000 ppm:

1, 3, 4.

Topical Application Tests

Blatella germanica

The activity of compounds of the invention against anaesthetised male Blatella germanica (WRL strain) was demonstrated by the topical application to the test insect of a solution of the compound under test in butanone. Mortality was assessed after 6 days.

The following compounds were active at less than 5μ/ins.:

11,14,12,13,16,17,20,22,23,24,27,28,33,41,40,35.

The following compounds were active at less than 1 μg/ins.

19, 25, 31, 34, 38.

Musca domestica topical application test.

The activity of compounds of the invention against anaesthetised female Musca domestica (WRL strain) was demonstrated by the topical application to the test insect of a solution of the compound under test in butanone. Mortality was assessed after 24 and 48 hours and included knockdown data.

The following compounds were active at less than 0.1μg/ins.:

16, 31, 38.

Periplaneta americana Topical application test.

The activity of compounds of the invention against anaesthetised male Periplaneta americana (WRL strain) was demonstrated by the topical application to the test insect of a solution of the compound under test in butanone. Mortality was assessed after 6 days.

The following compounds were active at less than 10 ppm.:

19, 31, 38.

Appendix 1

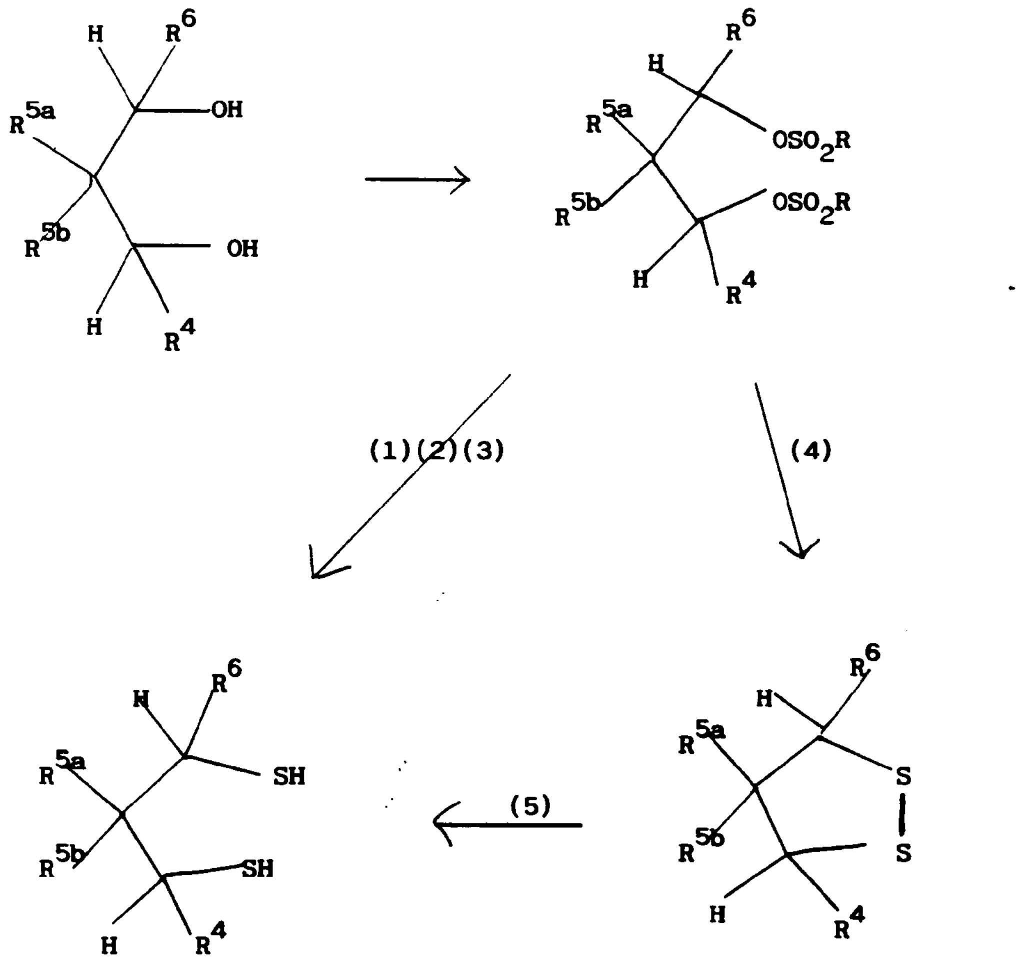


(1) $Na_2CS_3H_2O$ (2)HCl (3) $LiAlH_4$, $Et_2O$ (4) $Na_2S/S$, DMF (5) $LiAlH_4$,$Et_2O$

Appendix 2

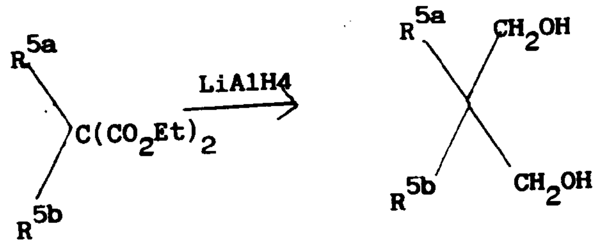

Appendix 3

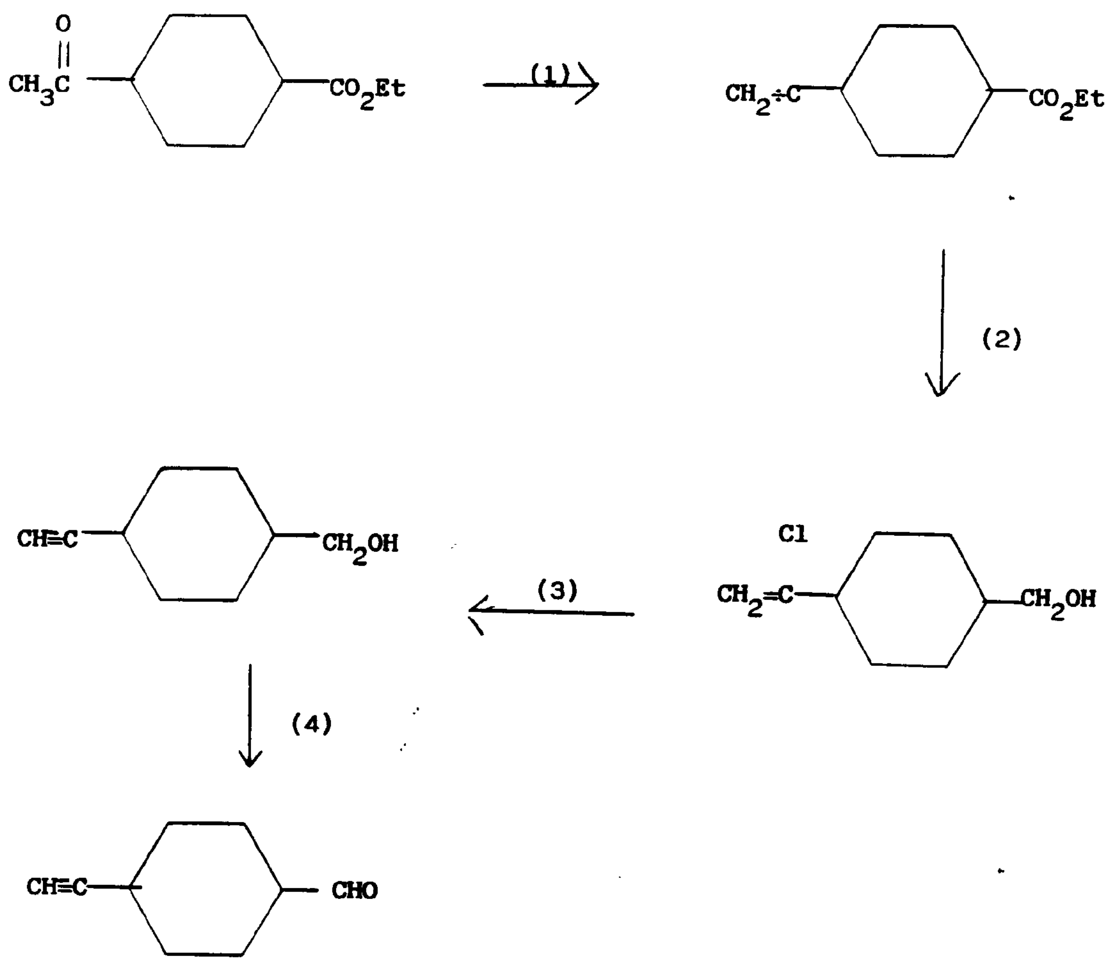


(1) $PCl_5$ pyridine (2) $LiAlH_4$, $Et_2O$ (3) n-BuLi, THF
(4) oxalyl chloride, $CH_2Cl_2$, DMSO, $NEt_3$

Formulations

| | |
|---|---|
| Compound of formula (I) | 10.00 |
| Alkyl phenol ethoxylate* | 7.50 |
| Alkyl aryl sulphonate* | 2.50 |
| $C_{8-13}$ aromatic solvent | 80.00 |
| | 100.00 |

| | |
|---|---|
| Compound of formula (I) | 10.00 |
| Alkyl phenol ethoxylate* | 2.50 |
| Alkyl aryl sulphonate* | 2.50 |
| Ketonic solvent | 64.00 |
| $C_{8-13}$ aromatic solvent | 18.00 |
| Antioxidant | 3.00 |
| | 100.00 |

| | |
|---|---|
| Compound of formula (I) | 5.00 |
| $C_{8-13}$ aromatic solvent | 7.00 |
| $C_{18}$ aromatic solvent | 28.00 |
| China clay | 10.00 |
| Alkyl aryl sulphonate* | 1.00 |
| Napthalene sulphonic acid* | 3.00 |
| Diatomaceous earth | 46.00 |
| | 100.00 |

| | |
|---|---|
| Compound of formula (I) | 0.50 |
| Talc | 99.50 |
| | 100.00 |

| | |
|---|---|
| Compound of formula (I) | 0.5 |
| Sugar | 79.5 |
| Paraffin wax | 20.0 |
| | 100.00 |

| | |
|---|---|
| Compound of formula (I) | 5.00 |
| $C_{8-13}$ aromatic solvent | 32.00 |
| Cetyl alcohol | 3.00 |
| Polyoxyethylene glycerol monooleate* | 0.75 |
| Polyoxyethylene sorbitan esters* | 0.25 |
| Silicone solution | 0.1 |
| Water | 58.9 |
| | 100.00 |

| | |
|---|---|
| Compound of formula (I) | 10.00 |
| Alkyl aryl ethoxylate* | 3.00 |
| Silicone solution | 0.1 |
| Alkane diol | 5.0 |
| Fumed silica | 0.50 |
| Xanthan gum | 0.20 |
| Water | 80.0 |
| Buffering agent | 1.2 |
| | 100.00 |

| | |
|---|---|
| Compound of formula (I) | 10.00 |
| Polyoxyethylene glycerol monooleate* | 10.00 |
| Alkane diol | 4.00 |
| Water | 76.00 |
| | 100.00 |

| | |
|---|---|
| Compound of formula (I) | 70.00 |
| Polyvinyl pyrrolidine | 2.50 |
| Alkyl aryl ethoxylate | 1.25 |
| Alkyl aryl sulphonate | 1.25 |
| China clay | 25.00 |
| | 100.00 |

| | |
|---|---|
| Compound of formula (I) | 2.00 |
| Alkyl phenol ethoxylate* | 5.00 |
| Alkyl aryl sulphonate* | 3.00 |
| $C_{8-13}$ aromatic solvent | 20.00 |
| Kieselguhr granules | |
| | 70.00 |
| | 100.00 |

| | |
|---|---|
| Compound of formula (I) | 0.3 |
| Piperonyl butoxide | 1.5 |
| $C_{8-13}$ saturated hydrocarbon solvent | 58.2 |
| Butane | 40.0 |
| | 100.00 |

| | |
|---|---|
| Compound of formula (I) | 0.3 |
| $C_{8-13}$ saturated hydrocarbon solvent | 10.0 |
| Sorbitan monooleate* | 1.0 |
| Water | 40.0 |
| Butane | 48.7 |
| | 100.00 |

| | |
|---|---|
| Compound of formula (I) | 1.00 |
| $CO_2$ | 3.00 |
| Polyoxyethylene glycerol monooleate* | 1.40 |
| Propanone | 38.00 |
| Water | 56.60 |
| | 100.00 |

| | |
|---|---|
| Compound of formula (I) | 2.50 |
| Resin | 5.00 |
| Antioxidant | 0.50 |
| High aromatic white spirit | 92.0 |
| | 100.00 |

| | |
|---|---|
| Compound of formula (I) | 0.10 |
| Antioxidant | 0.10 |
| Odourless kerosene | 99.8 |
| | 100.00 |

| | |
|---|---|
| Compound of formula (I) | 0.10 |
| Piperonyl butoxide | 0.50 |
| Antioxidant | 0.10 |
| Odourless kerosene | 99.30 |
| | 100.00 |

| | |
|---|---|
| Compound of formula (I) | 10.0 |
| $C_{8-13}$ aromatic solvent | 10.0 |
| Aromatic di-isocyanate# | 4.5 |
| Alkyl phenol ethoxylate* | 6.0 |
| Alkyl diamine# | 1.0 |
| Diethylene triamine | 1.0 |
| Concentrated hydrochloric acid | 2.2 |
| Xanthan gum | 0.2 |
| Fumed silica | 0.5 |
| Water | 64.6 |
| | 100.00 |

* = Surfactant

# = react to form the polyurea walls of the microcapsule

Antioxidant could be any of the following individually or combined

Butylated hydroxytoluene

Butylated hydroxyanisole

Vitamin C (ascrobic acid)

## Claims

### Claims for the following Contracting States : AT, BE, CH, DE, FR, GB, IT, LI, LU, NL, SE

1. A compound of the formula (I):

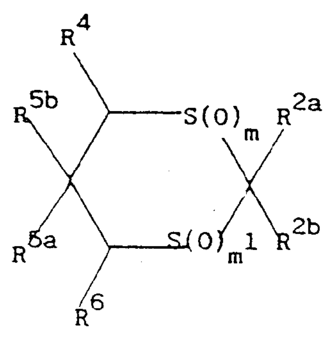

(I)

which contains between 9 and 27 carbon atoms, and wherein m and $m^1$ are independently selected from 0, 1 and 2; $R^{2a}$ is hydrogen, methyl, or ethyl; $R^{2b}$ is ethynyl or contains between 3 and 18 carbon atoms and is a group $R^7$, wherein $R^7$ is a $C_{1-13}$ non-aromatic hydrocarbyl group, optionally substituted by a cyano or $C_{1-4}$ carbalkoxy group and/or by one or two hydroxy groups and/or by one to five halo atoms which are the same or different and/or by one to three groups $R^8$ which are the same or different and each contains one to four hetero atoms, which are the same or different and are chosen from oxygen, sulphur, nitrogen and silicon, 1 to 10 carbon atoms and optionally 1 to 6 fluoro or chloro atoms or $R^{2b}$ is a 6-membered aromatic ring substituted by cyano and/or by one to three groups $R^{8a}$ and/or by a group -C≡CH, -C≡C-$R^{7a}$ or C≡C-halo and/or by one to five halo atoms and/or by one to three $C_{1-4}$ haloalkyl groups wherein $R^{7a}$ and $R^{8a}$ are groups $R^7$ and $R^8$ as hereinbefore defined; $R^4$ and $R^6$ are the same or different and are chosen from hydrogen, methyl, trifluoromethyl or cyano; $R^{5a}$ is a group

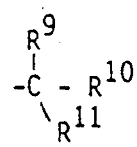

wherein $R^{11}$ is hydrogen, methyl, trifluoromethyl, iodo, fluoro, chloro or bromo, $R^9$ is methyl, ethyl, chloro, bromo, methoxy, cyano, nitro, methoxymethyl, $C_{1-4}$ carbalkoxy or trifluoromethyl, $R^{10}$ is chloro, methyl or trifluoromethyl, or the moiety

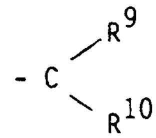

is a -C=$CR^{12}R^{13}$ group wherein $R^{12}$ and $R^{13}$ are both hydrogen, methyl, trifluoromethyl, fluoro, chloro or bromo or $R^{12}$ is hydrogen and $R^{13}$ is fluoro, chloro or bromo, or the moiety

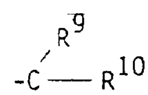

is a three or four membered ring,

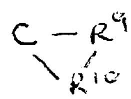

in which $R^9$ is oxygen or a group $CR^{14}$ $R^{15}$ wherein the groups $R^{14}$ and $R^{15}$ are the same or different and each is hydrogen, fluoro, chloro or bromo or methyl or ethyl optionally substituted by 1 to 3 fluoro atoms and in which $R^{10}$ is a group $CR^{14a}R^{15a}$ or a group $CR^{14a}R^{15a}CR^{16}R^{17}$ wherein $R^{14a}$, $R^{15a}$ are the same or different and each is hydrogen, fluoro, chloro or bromo or methyl or ethyl optionally substituted by 1 to 5 fluoro atoms, and $R^{16}$ and $R^{17}$ are the same or different and each is hydrogen, fluoro, chloro or bromo or methyl or ethyl optionally substituted by 1 to 5 fluoro atoms; and $R^{5b}$ is hydrogen or $C_{1-3}$ alkyl; provided that $R^{2b}$ is not propyl or butyl and that $R^{5a}$ is not tertiary butyl or tertiary amyl.

2. A compound of the formula (I) according to claim 1 in which $R^{2b}$ is a phenyl group substituted at the 3-,4- or 5-positions by one to three substituents each selected from halo, $C_{1-4}$ haloalkyl, $C_{1-4}$ haloalkoxy, $C_{1-4}$ haloalkylthio, cyano, or a group $(C{\equiv}C)_pR^{19}$ wherein p is 1 or 2 and $R^{19}$ is hydrogen, bromo, chloro, iodo or a group $S(O)_qR^{20}$ wherein q is 0, 1 or 2 and $R^{20}$ is trifluoromethyl, methyl or ethyl; or $R^{19}$ is an aliphatic group containing up to five carbon atoms optionally substituted by $C_{1-4}$ alkoxy, $C_{1-6}$ alkoxyalkoxy, $C_{1-8}$ acyloxy, halo or hydroxy or $R^{19}$ is a group $COR^{21}$ wherein $R^{21}$ is hydrogen, $C_{1-4}$ alkoxy, $C_{1-4}$ alkyl or a group $NR^{22}R^{23}$ wherein $R^{22}$ and $R^{23}$ are independently selected from hydrogen, methyl or ethyl; or $R^{19}$ is $SiR^{24}$, $R^{25}$, $R^{26}$ wherein $R^{24}$ and $R^{25}$ are the same or different and are each $C_{1-4}$ aliphatic groups and $R^{26}$ is a $C_{1-4}$ aliphatic group or phenyl provided that $R^{24}$, $R^{25}$, and $R^{26}$ do not contain more than 10 carbon atoms in total and the phenyl group is additionally optionally substituted at the 2- and/or 6-positions by fluoro or chloro.

3. A compound of the formula (I) according to claim 1 in which $R^{2b}$ is a group -A(C≡C)Z, wherein A is a $C_{3-5}$ aliphatic chain optionally containing a double bond and/or an oxygen atom and/or a group S(O)q wherein q is 0, 1 or 2 optionally substituted by halo, $C_{1-4}$ alkyl, $C_{1-4}$ haloalkyl, $C_{1-4}$ carbalkoxy or cyano and Z is hydrogen, $C_{1-5}$ alkyl, $C_{1-3}$ alkoxymethyl or a group $SiR^{24}$, $R^{25}$, $R^{26}$ wherein $R^{24}$, $R^{25}$ and $R^{26}$ are as hereinbefore defined.

4. A compound of the formula (I) according to claim 1 in which $R^{2b}$ is a group $-BZ^1$, wherein B is a group $-CH_2O-$ or $CH_2S(O)_q$ wherein q is 0, 1 or 2 or a $C_{2-3}$ aliphatic group each of which may be optionally substituted by one to three halo atoms and $Z^1$ is silyl substituted by three $C_{1-4}$ alkyl groups or $Z^1$ is a group

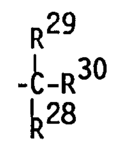

wherein $R^{28}$, $R^{29}$, and $R^{30}$ are the same or different and are each independently selected from halo, cyano, $C_{1-5}$ carbalkoxy, or a $C_{1-4}$ aliphatic group optionally substituted by halo,cyano, $C_{1-5}$ carbalkoxy, $C_{1-4}$ alkoxy or a group $S(O)_q$ $R^{31}$ wherein q is 0, 1 or 2 and $R^{31}$ is $C_{1-4}$ alkyl, or $R^{28}$, $R^{29}$, and $R^{30}$ are selected from $C_{1-4}$ alkoxy or a group $S(O)_wR^{32}$ wherein w is 0, 1 or 2 and $R^{32}$ is $C_{1-4}$ alkyl optionally substituted by fluoro or $R^{28}$ and $R^{29}$ are linked to form a $C_{3-6}$ cycloalkyl ring, or one of $R^{28}$, $R^{29}$ and $R^{30}$ may be hydrogen.

5. A compound of the formula (I) according to claim 1 in which $R^{2b}$ is a group

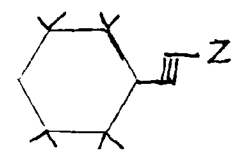

wherein Z is as hereinbefore defined:

6. A compound of the formula (I) according to any one of claims 1 to 5 in which $R^{2a}$ is hydrogen or methyl and $R^4$ and $R^6$ are hydrogen.

7. A compound of the formula (I) according to any one of claims 1 to 6 in which $R^{5a}$ is an isopropyl, isopropenyl cyclopropyl, methylcyclopropyl, cyclobutyl, 1-trifluoromethylethyl or 1,1-dimethyl-2,2,2-trifluoro ethyl group.

8. A compound of the formula (I) according to claim 7 in which $R^{5a}$ is an isopropyl group.

9. A compound of the formula (I) according to claim 1 selected from the group comprising :

cis/trans-2-(4-Bromophenyl)-5-isopropyl-1,3-dithiane.
cis/trans-5-Isopropyl-2-(4-trimethylsilylethynylphenyl)-1,3-dithiane.
cis/trans-2-(4-Ethynylphenyl)-5-isopropyl-1,3-dithiane.
trans-2-(4-Ethynylphenyl)-5-isopropyl-1,3-dithiane.
cis/trans-2-(4-Ethynylphenyl)-5-isopropyl-2-methyl-1,3-dithiane.
cis/trans-2-[4-(3-Acetoxyprop-1-ynyl)phenyl]-5-isopropyl-1,3-dithiane.
trans-2-[4-(3-Acetoxyprop-1-ynyl)phenyl]-5-isopropyl-1,3-dithiane.
trans-2-[4-(3-Benzoyloxyprop-1-ynyl)phenyl]-5-isopropyl-1,3-dithiane.
cis/trans-2-(trans-4-ethynylcyclohexyl)-5-isopropyl-1,3-dithiane.
cis/trans-2-(Hex-5-ynyl)-5-isopropyl-1,3-dithiane.
cis/trans-2-(trans-4-ethynylcyclohexyl)-5-isopropyl-1,3-dithiane oxide.
trans-2-(4-Bromophenyl)-5-isopropyl-1,3-dithiane 1,3-dioxide.
2(e)-(4-Ethynylphenyl)-5(e)-isopropyl-1,3-dithiane 1(e)-oxide.
cis-2(e)-(4-Ethynylphenyl)-5(a)-isopropyl-1,3-dithiane.
trans-2(e)-(trans-4(e)-Ethynylcyclohexyl)-5(e)-isopropyl-1,3-dithiane 1(e)-oxide.
trans-2(e)-[(E)-Hex-1-en-5-ynyl)-5(e)-isopropyl-1,3-dithiane.
cis/trans-5-Isopropyl-2-methyl-2-(3,3,3-trichlopropyl)-1,3-dithiane
cis/trans-2(e)-(4-Ethynylphenyl)-5-(1-trifluoromethylethyl)-1,3-dithiane
trans-2(e)-(4-Ethynylphenyl)-5(e)-(1-trifluoromethylethyl)-1,3-dithiane.
trans-2(e)-(trans-4(e)-Ethynylcyclohexyl)-5(e)-1-trifluoromethylethyl)-1,3-dithiane.
cis/trans-5(e)-(1,1-Dimethyl-2,2,2-trifluoroethyl)-2-(4-trimethylsilylethynylphenyl)-1,3-dithiane.
trans-5(e)-(1,1-Dimethyl-2,2,2-trifluoroethyl)-2(e)-(4-ethynylphenyl)-1,3-dithiane.
cis/trans-5(e)-(1,1-Dimethyl-2,2,2-trifluoroethyl)-2-(4-ethynylphenyl)-1,3-dithiane.
cis/trans-5-Cyclopropyl-2-(cis/trans-4-ethynylcyclohexyl)-1,3-dithiane.
trans-5(e)-Cyclopropyl-2(e)-(4-ethynylphenyl)-1,3-dithiane.
trans-5-Cyclobutyl-2-(4-ethynylphenyl)-1,3-dithiane.
trans-2-(4-Ethynylphenyl)-5-(1-methylcyclopropyl)-1,3-dithiane.
cis/trans-5-(1-Methylpropyl)-2-(4-ethynylphenyl)-1,3-dithiane.
trans-5(e)-(1-Methylpropyl)-2(e)-(4-trimethylsilylethynylphenyl)-1,3-dithiane.

10. A process for the preparation of a compound of the formula (I) according to any one of claims 1 to 9,

which comprises

i) the reaction of a compound of the formula (II):

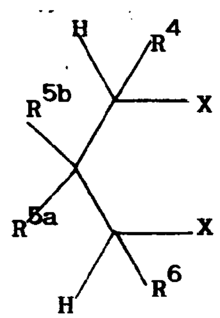

(II)

wherein X is SH with a suitable aldehyde or ketone of the formula

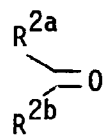

or a reactive derivative thereof, wherein $R^{2a}$, $R^{2b}$, $R^4$, $R^{5a}$, $R^{5b}$ and $R^6$ are as hereinbefore defined and, if required, thereafter oxidizing one or both of the ring sulphur atoms.

(ii) When $R^{2a}$ is hydrogen, the reaction of a dithiaborinane-dimethylsulphide complex of a compound of the formula (II) with a carboxylic acid

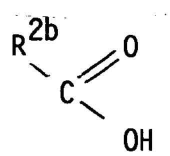

This reaction is carried out in the presence of a agent such as stannous chloride in an inert solvent such as an ether, conveniently tetrahydrofuran, at a non-extreme temperature, for example between -20° and 100° and conveniently between 10° and 30°,

and thereafter, if desired, converting one compound of the formula (I) to another compound of the formula (I) by methods well known to those skilled in the art.

11. An insecticidal or acaricidal composition comprising a compound of formula (I) as defined in any one of claims 1-9 in admixture with a carrier or diluent.

12. A synergised pesticidal composition comprising a compound of formula (I), as defined in any one of claims 1-9, a synergist for the formula (I) compound and a carrier or diluent.

13. A mixture of a compound of formula (I) as defined in any one of claims 1-9 and another pesticidal compound.

14. A method for the control of pests comprising application to the pest or to an environment susceptible to pest infestation of a pesticidally effective amount of a compound according to any one of claims 1-9 or a composition or mixture according to any one of claims 11-13.

15. A compound as defined in any one of claims 1-9 or composition according to any one of claims 11-13 for use in a method of surgery or therapy practised on the human or animal body or in a method of diagnosis practised on the human or animal body.

**Claims for the following Contracting States : ES, GR**

1. A process for the preparation of a compound of the formula (I):

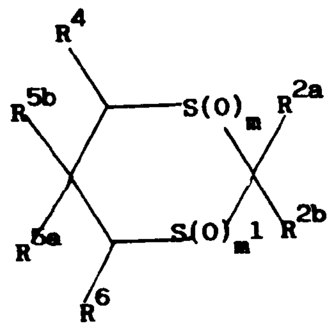

(I)

which contains between 9 and 27 carbon atoms, and wherein m and $m^1$ are independently selected from 0, 1 and 2; $R^{2a}$ is hydrogen, methyl, or ethyl; $R^{2b}$ is ethynyl or contains between 3 and 18 carbon atoms and is a group $R^7$, wherein $R^7$ is a $C_{1-13}$ non-aromatic hydrocarbyl group, optionally substituted by a cyano or $C_{1-4}$ carbalkoxy group and/or by one or two hydroxy groups and/or by one to five halo atoms which are the same or different and/or by one to three groups $R^8$ which are the same or different and each contains one to four hetero atoms, which are the same or different and are chosen from oxygen, sulphur, nitrogen and silicon, 1 to 10 carbon atoms and optionally 1 to 6 fluoro or chloro atoms or $R^{2b}$ is a 6-membered aromatic ring substituted by cyano and/or by one to three groups $R^{8a}$ and/or by a group -C≡CH, -C≡C-$R^{7a}$ or C≡C-halo and/or by one to five halo atoms and/or by one to three $C_{1-4}$ haloalkyl groups wherein $R^{7a}$ and $R^{8a}$ are groups $R^7$ and $R^8$ as hereinbefore defined; $R^4$ and $R^6$ are the same or different and are chosen from hydrogen, methyl, trifluoromethyl or cyano; $R^{5a}$ is a group

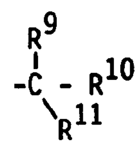

wherein $R^{11}$ is hydrogen, methyl, trifluoromethyl, iodo, fluoro, chloro or bromo, $R^9$ is methyl, ethyl, chloro, bromo, methoxy, cyano, nitro, methoxymethyl, $C_{1-4}$ carbalkoxy or trifluoromethyl, $R^{10}$ is chloro, methyl or trifluoromethyl, or the moiety

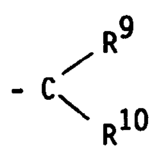

is a -C=$CR^{12}R^{13}$ group wherein $R^{12}$ and $R^{13}$ are both hydrogen, methyl, trifluoromethyl, fluoro, chloro or bromo, or $R^{12}$ is hydrogen and $R^{13}$ is fluoro, chloro or bromo, or the moiety

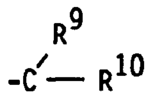

is a three or four membered ring,

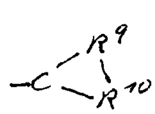

in which

$R^9$ is oxygen or a group $CR^{14}R^{15}$ wherein the groups $R^{14}$ and $R^{15}$ are the same or different and each is hydrogen,

fluoro, chloro or bromo or methyl or ethyl optionally substituted by 1 to 3 fluoro atoms, and in which $R^{10}$ is a group $CR^{14a}R^{15a}$ or a group $CR^{14a}R^{15a}CR^{16}R^{17}$ wherein $R^{14a}$,$R^{15a}$ are the same or different and each is hydrogen, fluoro, chloro or bromo or methyl or ethyl optionally substituted by 1 to 5 fluoro atoms, and $R^{16}$ and $R^{17}$ are the same or different and each is hydrogen, fluoro, chloro or bromo or methyl or ethyl optionally substituted by 1 to 5 fluoro atoms; and $R^{5b}$ is hydrogen or $C_{1-3}$ alkyl; provided that $R^{2b}$ is not propyl or butyl and that $R^{5a}$ is not tertiary butyl or tertiary amyl, which process comprises:

i) the reaction of a compound of the formula (II):

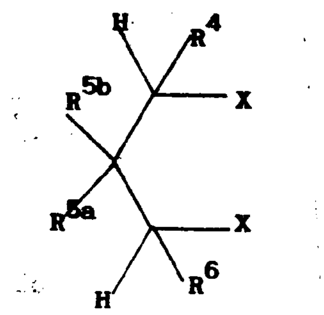

(II)

wherein X is SH with a suitable aldehyde or ketone of the formula

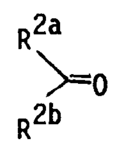

or a reactive derivative thereof, wherein $R^{2a}$, $R^{2b}$, $R^4$, $R^{5a}$, $R^{5b}$ and $R^6$ are as hereinbefore defined and, if required, thereafter oxidizing one or both of the ring sulphur atoms.

(ii) When $R^{2a}$ is hydrogen, the reaction of a dithiaborinane-dimethylsulphide complex of a compound of the formula (II) with a carboxylic acid

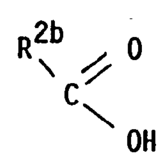

This reaction is carried out in the presence of a agent such as stannous chloride in an inert solvent such as an ether, conveniently tetrahydrofuran, at a non-extreme temperature, for example between -20° and 100° and conveniently between 10° and 30°,

and thereafter, if desired, converting one compound of the formula (I) to another compound of the formula (I) by methods well known to those skilled in the art.

2. A process for the preparation of a compound of the formula (I) according to claim 1 in which $R^{2b}$ is a phenyl group substituted at the 3- ,4- or 5-positions by one to three substituents each selected from halo, $C_{1-4}$ haloalkyl, $C_{1-4}$ haloalkoxy, $C_{1-4}$ haloalkylthio, cyano, or a group $(C{\equiv}C)_pR^{19}$ wherein p is 1 or 2 and $R^{19}$ is hydrogen, bromo, chloro, iodo or a group $S(O)_qR^{20}$ wherein q is 0, 1 or 2 and $R^{20}$ is trifluoromethyl, methyl or ethyl; or $R^{19}$ is an aliphatic group containing up to five carbon atoms optionally substituted by $C_{1-4}$ alkoxy, $C_{1-6}$ alkoxyalkoxy, $C_{1-8}$ acyloxy, halo or hydroxy or $R^{19}$ is a group $COR^{21}$ wherein $R^{21}$ is hydrogen, $C_{1-4}$ alkoxy, $C_{1-4}$ alkyl or a group $NR^{22}R^{23}$ wherein $R^{22}$ and $R^{23}$ are independently selected from hydrogen, methyl or ethyl; or $R^{19}$ is $SiR^{24}$, $R^{25}$, $R^{26}$ wherein $R^{24}$ and $R^{25}$ are the same or different and are each $C_{1-4}$ aliphatic groups and $R^{26}$ is a $C_{1-4}$ aliphatic group or phenyl provided that $R^{24}$, $R^{25}$ and $R^{26}$ do not contain more than 10 carbon atoms in total and the phenyl group is additionally optionally substituted at the 2- and/or 6-positions by fluoro or chloro.

3. A process for the preparation of a compound of the formula (I) according to claim 1 in which $R^{2b}$ is a group $-A(C{\equiv}C)Z$, wherein A is a $C_{3-5}$ aliphatic chain optionally containing a double bond and/or an oxygen atom and/or a group S(O)q wherein q is 0, 1 or 2 optionally substituted by halo, $C_{1-4}$ alkyl, $C_{1-4}$ haloalkyl, $C_{1-4}$ car-

balkoxy or cyano and Z is hydrogen, $C_{1-5}$ alkyl, $C_{1-3}$ alkoxymethyl or a group $SiR^{24},R^{25},R^{26}$ wherein $R^{24}$, $R^{25}$ and $R^{26}$ are as hereinbefore defined.

4. A process for the preparation of a compound of the formula (I) according to claim 1 in which $R^{2b}$ is a group $-BZ^1$, wherein B is a group $-CH_2O-$ or $CH_2S(O)q$ wherein q is 0, 1 or 2 or a $C_{2-3}$ aliphatic group each of which may be optionally substituted by one to three halo atoms and $Z^1$ is silyl substituted by three $C_{1-4}$ alkyl groups or $Z^1$ is a group

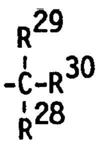

wherein $R^{28}$, $R^{29}$ and $R^{30}$ are the same or different and are each independently selected from halo, cyano, $C_{1-5}$ carbalkoxy, or a $C_{1-4}$ aliphatic group optionally substituted by halo,cyano, $C_{1-5}$ carbalkoxy, $C_{1-4}$ alkoxy or a group $S(O)_q$ $R^{31}$ wherein q is 0, 1 or 2 and $R^{31}$ is $C_{1-4}$ alkyl, or $R^{28}$, $R^{29}$ and $R^{30}$ are selected from $C_{1-4}$ alkoxy or a group $S(O)_wR^{32}$ wherein w is 0, 1 or 2 and $R^{32}$ is $C_{1-4}$ alkyl optionally substituted by fluoro or $R^{28}$ and $R^{29}$ are linked to form a $C_{3-6}$ cycloalkyl ring, or one of $R^{28}$, $R^{29}$ and $R^{30}$ may be hydrogen.

5. A process for the preparation of a compound of the formula (I) according to claim 1 in which $R^{2b}$ is a group

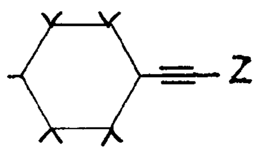

wherein Z is as hereinbefore defined:

6. A process for the preparation of a compound of the formula (I) according to any one of claims 1 to 5 in which $R^{2a}$ is hydrogen or methyl and $R^4$ and $R^6$ are hydrogen.

7. A process for the preparation of a compound of the formula (I) according to any one of claims 1 to 6 in which $R^{5a}$ is an isopropyl, isopropenyl cyclopropyl, methylcyclopropyl, cyclobutyl, 1-trifluoromethylethyl or 1,1-dimethyl-2,2,2-trifluoro ethyl group.

8. A process for the preparation of a compound of the formula (I) according to claim 7 in which $R^{5a}$ is an isopropyl group.

9. A process for the preparation of a compound of the formula (I) according to any one of claims 1 to 8 selected from the group comprising :
cis/trans-2-(4-Bromophenyl)-5-isopropyl-1,3-dithiane.
cis/trans-5-Isopropyl-2-(4-trimethylsilylethynylphenyl)-1,3-dithiane.
cis/trans-2-(4-Ethynylphenyl)-5-isopropyl-1,3-dithiane.
trans-2-(4-Ethynylphenyl)-5-isopropyl-1,3-dithiane.
cis/trans-2-(4-Ethynylphenyl)-5-isopropyl-2-methyl-1,3-dithiane.
cis/trans-2-[4-(3-Acetoxyprop-1-ynyl)phenyl]-5-isopropyl-1,3-dithiane.
trans-2-[4-(3-Acetoxyprop-1-ynyl)phenyl]-5-isopropyl-1,3-dithiane.
trans-2-[4-(3-Benzoyloxyprop-1-ynyl)phenyl]-5-isopropyl-1,3-dithiane.
cis/trans-2-(trans-4-ethynylcyclohexyl)-5-isopropyl-1,3-dithiane.
cis/trans-2-(Hex-5-ynyl)-5-isopropyl-1,3-dithiane.
cis/trans-2-(trans-4-ethynylcyclohexyl)-5-isopropyl-1,3-dithiane oxide.
trans-2-(4-Bromophenyl)-5-isopropyl-1,3-dithiane 1,3-dioxide.
2(e)-(4-Ethynylphenyl)-5(e)-isopropyl-1,3-dithiane 1(e)-oxide.
cis-2(e)-(4-Ethynylphenyl)-5(a)-isopropyl-1,3-dithiane.
trans-2(e)-(trans-4(e)-Ethynylcyclohexyl)-5(e)-isopropyl-1,3-dithiane 1(e)-oxide.
trans-2(e)-[(E)-Hex-1-en-5-ynyl)-5(e)-isopropyl-1,3-dithiane.
cis/trans-5-Isopropyl-2-methyl-2-(3,3,3-trichlolpropyl)-1,3-dithiane
cis/trans-2(e)-(4-Ethynylphenyl)-5-(1-trifluoromethylethyl)-1,3-dithiane
trans-2(e)-(4-Ethynylphenyl)-5(e)-(1-trifluoromethylethyl)-1,3-dithiane.

trans-2(e)-(trans-4(e)-Ethynylcyclohexyl)-5(e)-1-trifluoromethylethyl)-1,3-dithiane.
cis/trans-5(e)-(1,1-Dimethyl-2,2,2-trifluoroethyl)-2-(4-trimethylsilylethynylphenyl)-1,3-dithiane.
trans-5(e)-(1,1-Dimethyl-2,2,2-trifluoroethyl)-2(e)-(4-ethynylphenyl)-1,3-dithiane.
cis/trans-5(e)-(1,1-Dimethyl-2,2,2-trifluoroethyl)-2-(4-ethynylphenyl)-1,3-dithiane.
cis/trans-5-Cyclopropyl-2-(cis/trans-4-ethynylcyclohexyl)-1,3-dithiane.
trans-5(e)-Cyclopropyl-2(e)-(4-ethynylphenyl)-1,3-dithiane.
trans-5-Cyclobutyl-2-(4-ethynylphenyl)-1,3-dithiane.
trans-2-(4-Ethynylphenyl)-5-(1-methylcyclopropyl)-1,3-dithiane.
cis/trans-5-(1-Methylpropyl)-2-(4-ethynylphenyl)-1,3-dithiane.
trans-5(e)-(1-Methylpropyl)-2(e)-(4-trimethylsilylethynylphenyl)-1,3-dithiane.

10. An insecticidal or acaricidal composition comprising a compound of formula (I) as defined in any one of claims 1-8 in admixture with a carrier or diluent.

11. A synergised pesticidal composition comprising a compound of formula (I), as defined in any one of claims 1-8, a synergist for the formula (I) compound and a carrier or diluent.

12. A mixture of a compound of formula (I) as defined in any one of claims 1-8 and another pesticidal compound.

13. A method for the control of pests comprising application to the pest or to an environment susceptible to pest infestation of a pesticidally effective amount of a compound according to any one of claims 1-8 or a composition or mixture according to any one of claims 10-12.

14. A compound as defined in any one of claims 1-9 or composition according to any one of claims 10-12 for use in a method of surgery or therapy practised on the human or animal body or in a method of diagnosis practised on the human or animal body.

## Patentansprüche

### Patentansprüche für folgende Vertragsstaaten : AT, BE, CH, DE, FR, GB, IT, LI, LU, NL, SE

1. Verbindung der Formel (I):

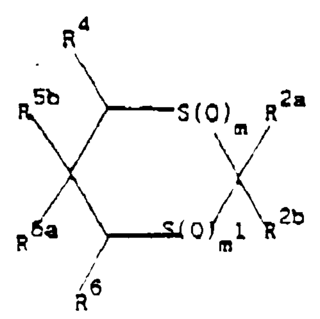

die zwischen 9 und 27 Kohlenstoffatome aufweist und worin m und $m^1$ unabhängig 0, 1 oder 2 sind; $R^{2a}$ Wasserstoff, Methyl oder Ethyl bedeutet; $R^{2b}$ Ethinyl bedeutet oder zwischen 3 und 18 Kohlenstoffatome aufweist und eine Gruppe $R^7$ bedeutet, worin $R^7$ eine nicht aromatische, gegebenenfalls durch Cyan oder eine $C_{1-4}$-Carbalkoxygruppe und/oder durch eine oder zwei Hydroxylgruppen und/oder durch ein bis fünf Halogenatome, die gleich oder verschieden sind, und/oder durch eine bis drei $R^8$-Gruppen, die gleich oder verschieden sind und jeweils ein bis vier Heteroatome, die gleich oder verschieden und aus Sauerstoff, Schwefel, Stickstoff und Silizium ausgewählt sind, 1 bis 10 Kohlenstoffatome und gegebenenfalls 1 bis 6 Fluor- oder Chloratome aufweisen, substituierte $C_{1-13}$-Kohlenwasserstoffgruppe, oder $R^{2b}$ einen durch Cyan und/oder durch eine bis drei $R^{8a}$-Gruppen und/oder durch eine Gruppe -C≡CH, -C≡C-$R^{7a}$ oder C≡C-Halogen und/oder durch ein bis fünf Halogenatome und/oder durch ein bis drei $C_{1-4}$-Halogenalkylgruppen, worin $R^{7a}$ und $R^{8a}$ wie zuvor definierte $R^7$- und $R^8$-Gruppen darstellen, substituierten aromatischen Sechsring bedeutet; $R^4$ und $R^6$ gleich oder verschieden und ausgewählt sind aus Wasserstoff, Methyl, Trifluormethyl oder Cyan; $R^{5a}$ stellt eine Gruppe

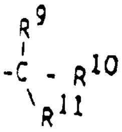

dar, worin $R^{11}$ Wasserstoff, Methyl, Trifluormethyl, Jod, Fluor, Chlor oder Brom, $R^9$ Methyl, Ethyl, Chlor, Brom, Methoxy, Cyan, Nitro, Methoxymethyl, $C_{1-4}$-Carbalkoxy oder Trifluormethyl, $R^{10}$ Chlor, Methyl oder Trifluormethyl bedeuten, oder der Molekülteil

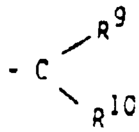

eine -C=$CR^{12}R^{13}$-Gruppe bedeutet, worin $R^{12}$ und $R^{13}$ jeweils für Wasserstoff, Methyl, Trifluormethyl, Fluor, Chlor oder Brom oder $R^{12}$ für Wasserstoff und $R^{13}$ für Fluor, Chlor oder Brom stehen, oder der Molekülteil

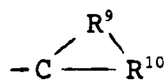

einen Drei- oder Vierring

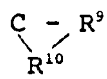

bedeutet, worin $R^9$ Sauerstoff oder eine $CR^{14}R^{15}$-Gruppe, worin die Gruppen $R^{14}$ und $R^{15}$ gleich oder verschieden sind und jeweils Wasserstoff, Fluor, Chlor oder Brom oder Methyl oder Ethyl, das gegebenenfalls durch 1 bis 3 Fluoratome substituiert ist und worin $R^{10}$ eine $CR^{14a}R^{15a}$-Gruppe oder eine $CR^{14a}R^{15a}CR^{16}R^{17}$-Gruppe, worin $R^{14a}$, $R^{15a}$ gleich oder verschieden sind und jeweils Wasserstoff, Fluor, Chlor oder Brom oder Methyl oder Ethyl, das gegebenenfalls durch 1 bis 5 Fluoratome substituiert ist, sowie $R^{16}$ und $R^{17}$ gleich oder verschieden sind und jeweils Wasserstoff, Fluor, Chlor oder Brom oder Methyl oder Ethyl, das gegebenenfalls durch 1 bis 5 Fluoratome substituiert ist; und $R^{5b}$ Wasserstoff oder $C_{1-3}$-Alkyl bedeuten; vorausgesetzt, daß $R^{2b}$ weder Propyl noch Butyl bedeutet und daß $R^{5a}$ weder tertiäres Butyl noch tertiäres Amyl bedeutet.

2. Verbindung der Formel (I) nach Anspruch 1, worin $R^{2b}$ eine in der 3-, 4- oder 5-Stellung durch jeweils aus Halogen,$C_{1-4}$-Halogenalkyl,$C_{1-4}$-Halogenalkoxy,$C_{1-4}$-Halogen- alkylthio, Cyan ausgewählte Substituenten einfach bis dreifach substituierte Phenylgruppe oder eine Gruppe $(C{\equiv}C)_pR^{19}$ bedeutet, worin p 1 oder 2 und $R^{19}$ Wasserstoff, Brom, Chlor, Jod oder eine Gruppe $S(O)_qR^{20}$ bedeuten, worin q 0, 1 oder 2 und $R^{20}$ Trifluormethyl, Methyl oder Ethyl; oder $R^{19}$ eine gegebenenfalls durch $C_{1-4}$-Alkoxy, $C_{1-6}$-Alkoxyalkoxy, $C_{1-8}$-Acyloxy, Halogen oder Hydroxyl substituierte aliphatische Gruppe mit bis zu fünf Kohlenstoffatomen, oder $R^{19}$ eine $COR^{21}$-Gruppe, worin $R^{21}$ Wasserstoff, $C_{1-4}$-Alkoxy, $C_{1-4}$-Alkyl oder eine $NR^{22}R^{23}$-Gruppe, worin $R^{22}$ und $R^{23}$ unabhängig aus Wasserstoff, Methyl oder Ethyl ausgewählt sind; oder $R^{19}$ $SiR^{24}R^{25}R^{26}$, worin $R^{24}$ und $R^{25}$ gleich oder verschieden sind und jeweils $C_{1-4}$-aliphatische Gruppen und $R^{26}$ eine $C_{1-4}$-aliphatische Gruppe oder Phenyl bedeuten, vorausgesetzt, daß $R^{24}$, $R^{25}$ und $R^{26}$ insgesamt nicht mehr als 10 Kohlenstoffatome aufweisen und die Phenylgruppe gegebenenfalls in der 2- und/oder 6-Stellung durch Fluor oder Chlor weiter substituiert ist.

3. Verbindung der Formel (I) nach Anspruch 1, worin $R^{2b}$ eine Gruppe $-A(C{\equiv}C)Z$, worin A eine $C_{3-5}$-aliphatische Kette darstellt, die gegebenenfalls eine Doppelbindung und/oder ein Sauerstoffatom und/ oder eine S(O)q-Gruppe aufweist, worin q 0, 1 oder 2 bedeutet, und gegebenenfalls durch Halogen, $C_{1-4}$-Alkyl, $C_{1-4}$-Halogenalkyl, $C_{1-4}$-Carbalkoxy oder Cyan substituiert ist, und Z Wasserstoff, $C_{1-5}$-Alkyl, $C_{1-3}$-Alkoxymethyl oder eine $SiR^{24}R^{25}R^{26}$-Gruppe bedeuten, worin $R^{24}$, $R^{25}$ und $R^{26}$ wie oben angegeben definiert sind.

4. Verbindung der Formel (I) nach Anspruch 1, worin $R^{2b}$ eine Gruppe $-BZ^I$, worin B eine Gruppe $-CH_2O$- oder $CH_2S(O)q$ darstellt, worin q 0, 1 oder 2, oder eine $C_{2-3}$-aliphatische Gruppe, die jeweils gegebenenfalls durch ein bis drei Halogenatome substituiert sind, sowie $Z^1$ durch drei $C_{1-4}$-Alkylgruppen substituiertes Silyl

oder $Z^1$ eine Gruppe

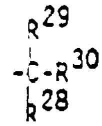

worin $R^{28}$, $R^{29}$ und $R^{30}$ gleich oder verschieden sind und jeweils unabhängig aus Halogen, Cyan, $C_{1-5}$-Carbalkoxy ausgewählt sind, oder eine gegebenenfalls durch Halogen, Cyan, $C_{1-5}$-Carbalkoxy, $C_{1-4}$-Alkoxy oder eine S(O)q$R^{31}$-Gruppe, worin q 0, 1 oder 2 und $R^{31}$ $C_{1-4}$-Alkyl ist, substituierte $C_{1-4}$-aliphatische Gruppe, oder $R^{28}$, $R^{29}$ und $R^{30}$ aus $C_{1-4}$-Alkoxy oder einer $S(O)_wR^{32}$-Gruppe ausgewählt sind, worin w 0, 1 oder 2 ist und $R^{32}$ gegebenenfalls durch Fluor substituiertes $C_{1-4}$-Alkyl bedeuten, oder $R^{28}$ und $R^{29}$ unter Bildung eines $C_{3-6}$-Cycloalkylrings verknüpft sind, oder einer der Reste $R^{28}$, $R^{29}$ und $R^{30}$ Wasserstoff bedeuten kann.

5. Verbindung der Formel (I) nach Anspruch 1, worin $R^{2b}$ eine Gruppe

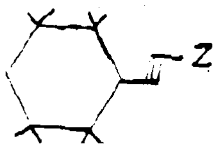

bedeutet, worin Z wie zuvor definiert ist.

6. Verbindung der Formel (I) nach einem der Ansprüche 1 bis 5, worin $R^{2a}$ Wasserstoff oder Methyl und $R^4$ und $R^6$ Wasserstoff bedeuten.

7. Verbindung der Formel (I) nach einem der Ansprüche 1 bis 6, worin $R^{5a}$ eine Isopropyl-, Isopropenyl-, Cyclopropyl-, Methylcyclopropyl-, Cyclobutyl, 1-Trifluormethylethyl- oder 1,1-Dimethyl-2,2,2-trifluorethylgruppe bedeutet.

8. Verbindung der Formel (I) nach Anspruch 7, worin $R^{5a}$ eine Isopropylgruppe bedeutet.

9. Verbindung der Formel (I) nach Anspruch 1, ausgewählt aus der Gruppe:
cis/trans-2-(4-Bromphenyl)-5-isopropyl-1,3-dithian,
cis/trans-5-isopropyl-2-(4-trimethylsilylethinylphenyl)1,3-dithian,
cis/trans-2-(4-Ethinylphenyl)-5-isopropyl-1,3-dithian,
trans-2-(4-Ethinylphenyl)-5-isopropyl-1,3-dithian,
cis/trans-2-(4-Ethinylphenyl)-5-isopropyl-2-methyl-1,3-dithian,
cis/trans-2-[4-(3-Acetoxyprop-1-inyl)phenyl]-5-isopropyl-1,3-dithian,
trans-2-[4-(3-Acetoxyprop-1-inyl)phenyl]-5-isopropyl-1,3-dithian,
trans-2-(4-(3-Benzoyloxyprop-1-inyl)phenyl]-5-isopropyl-1,3-dithian,
cis/trans-2-(trans-4-Ethinylcyclohexyl)-5-isopropyl-1,3-dithian,
cis/trans-2-(Hex-5-inyl)-5-isopropyl-1,3-dithian,
cis/trans-2-(trans-4-Ethinylcyclohexyl)-5-isopropyl-1,3-dithianoxid,
trans-2-(4-Bromphenyl)-5-isopropyl-1,3-dithian-1,3-dioxid,
2(e)-(4-Ethinylphenyl)-5(e)-isopropyl-1,3dithian-1(e)-oxid,
cis-2(e)-(4-Ethinylphenyl)-5(a)-isopropyl-1,3-dithian,
trans-2(e)-(trans-4(e)-Ethinylcyclohexyl)-5(e)-isopropyl-1,3-dithian-1(e)-oxid,
trans-2(e)-[(E)-Hex-1-en-5-inyl)-5(e)-isopropyl-1,3-dithian,
cis/trans-5-isopropyl-2-methyl-2-(3,3,3-trichlorpropyl)-1,3-dithian,
cis/trans-2(e)-(4-Ethinylphenyl)-5-(1-trifluormethylethyl)-1,3-dithian,
trans-2(e)-(4-Ethinylphenyl)-5(e)-(1-trifluormethylethyl)-1,3-dithian,
trans-2(e)-(trans-4(e)-Ethinylcyclohexyl)-5(e)-(1-trifluormethylethyl)-1,3-dithian,
cis/trans-5(e)-(1,1-Dimethyl-2,2,2-trifluorethyl)-2-(4-trimethylsilylethinylphenyl)-1,3-dithian,
trans-5(e)-(1,1-Dimethyl-2,2,2-trifluorethyl)-2(e)-(4-ethinylphenyl)-1,3-dithian,
cis/trans-5(e)-(1,1-Dimethyl-2,2,2-trifluorethyl)-2-(4-ethinylphenyl)-1,3-dithian,
cis/trans-5-Cyclopropyl-2-(cis/trans-4-ethinylcyclohexyl)-1,3-dithian,
trans-5(e)-Cyclopropyl-2(e)-(4-ethinylphenyl)-1,3-dithian,
trans-5-Cyclobutyl-2-(4-ethinylphenyl)-1,3-dithian,
trans-2-(4-Ethinylphenyl)-5-(1-methylcyclopropyl)-1,3-dithian,

cis/trans-5-(1-Methylpropyl)-2-(4-ethinylphenyl)-1,3-dithian,
trans-5(e)-(1-Methylpropyl)-2(e)-(4-trimethylsilylethinylphenyl)-1,3-dithian.

10. Verfahren zur Herstellung einer Verbindung der Formel (I) nach einem der Ansprüche 1 bis 9, das darin besteht, daß man

i) eine Verbindung der Formel (II):

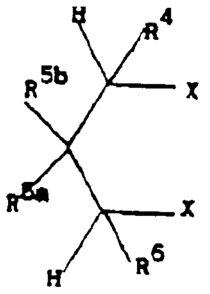

(II)

worin X SH bedeutet, mit einem geeigneten Aldehyd oder Keton der Formel

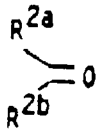

oder mit einem reaktiven Derivat davon, worin $R^{2a}$, $R^{2b}$ $R^4$ $R^{5a}$, $R^{5b}$ und $R^6$ wie oben angegeben definiert sind, umsetzt und anschließend gegebenenfalls eines oder beide der Ringschwefelatome oxidiert,
(ii) wenn $R^{2a}$ Wasserstoff bedeutet, einen Dithiaborinandimethylsulfidkomplex einer Verbindung der Formel (II) mit einer Carbonsäure

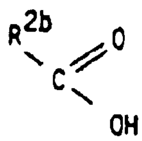

umsetzt, wobei diese Umsetzung in Anwesenheit eines Mittels wie z.B. Sn(II)-Chlorid in einem inerten Lösungsmittel wie einem Ether, zweckmäßig Tetrahydrofuran, bei einer nicht extremen Temperatur, zum Beispiel zwischen -20° und 100° und zweckmäßig zwischen 10° und 30° erfolgt,
und man anschließend gegebenenfalls eine Verbindung der Formel (I) mit Hilfe von dem Fachman bekannten Verfahren zu einer anderen Verbindung der Formel (I) umsetzt.

11. Insektizide oder akarazide Zusammensetzung aus einer gemäß einem der Ansprüche 1-9 definierten Verbindung der Formel (1) in einem Gemisch mit einem Träger oder Verdünnungsmittel.

12. Synergisierte schädlingsbekämpfende Zusammensetzung mit einer gemäß einem der Ansprüche 1-9 definierten Verbindung der Formel (I), einem Synergisten für die Verbindung der Formel (I) und einem Träger oder Verdünnungsmittel.

13. Gemisch aus einer gemäß einem der Ansprüche 1-9 definierten Verbindung der Formel (I) und einer weiteren schädlingsbekämpfenden Verbindung.

14. Verfahren zur Schädlingsbekämpfung, das darin besteht, daß man eine zur Schädlingsbekämpfung wirksame Menge einer Verbindung nach einem der Ansprüche 1-9 oder einer Zusammensetzung oder eines Gemisches nach einem der Ansprüche 11-13 auf die Schädlinge oder eine vom Schädlingsbefall bedrohte Fläche einwirken läßt.

15. Verbindung gemäß einem der Ansprüche 1-9 oder Zusammensetzung nach einem der Ansprüche 11-13 zur Verwendung in einem Verfahren zur chirurgischen oder therapeutischen Behandlung des menschlichen oder tierischen Körpers oder in einem Diagnostizierverfahren, das am menschlichen oder tierischen Körper vorgenommen wird.

**Patentansprüche für folgende Vertragsstaaten : ES, GR**

1. Verfahren zur Herstellung einer Verbindung der Formel (I):

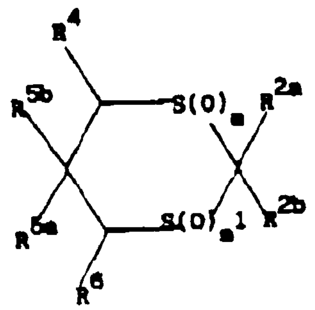

(I)

die zwischen 9 und 27 Kohlenstoffatome aufweist und worin m und $m^1$ unabhängig 0, 1 oder 2 sind; $R^{2a}$ Wasserstoff, Methyl oder Ethyl bedeutet; $R^{2b}$ Ethinyl bedeutet oder zwischen 3 und 18 Kohlenstoffatome aufweist und eine Gruppe $R^7$ bedeutet, worin $R^7$ eine nicht aromatische, gegebenenfalls durch Cyan oder eine $C_{1-4}$-Carbalkoxygruppe und/oder durch eine oder zwei Hydroxylgruppen und/oder durch ein bis fünf Halogenatome, die gleich oder verschieden sind, und/oder durch eine bis drei $R^8$-Gruppen, die gleich oder verschieden sind und jeweils ein bis vier Heteroatome, die gleich oder verschieden und aus Sauerstoff, Schwefel, Stickstoff und Silizium ausgewählt sind, 1 bis 10 Kohlenstoffatome und gegebenenfalls 1 bis 6 Fluor- oder Chloratome aufweisen, substituierte $C_{1-13}$-Kohlenwasserstoffgruppe, oder $R^{2b}$ einen durch Cyan und/oder durch eine bis drei $R^{8a}$-Gruppen und/oder durch eine -C≡CH-, -C≡C-$R^{7a}$- oder C≡C-Halogengruppe und/oder durch ein bis fünf Halogenatome und/oder durch ein bis drei $C_{1-4}$-Halogenalkylgruppen, worin $R^{7a}$ und $R^{8a}$ wie zuvor definierte $R^7$- und $R^8$-Gruppen darstellen, substituierten aromatischen Sechsring bedeutet; $R^4$ und $R^6$ gleich oder verschieden und ausgewählt sind aus Wasserstoff, Methyl, Trifluormethyl oder Cyan; $R^{5a}$ stellt eine Gruppe

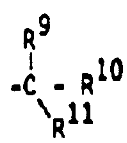

dar, worin $R^{11}$ Wasserstoff, Methyl, Trifluormethyl, Jod, Fluor, Chlor oder Brom, $R^9$ Methyl, Ethyl, Chlor, Brom, Methoxy, Cyan, Nitro, Methoxymethyl, $C_{1-4}$-Carbalkoxy oder Trifluormethyl, $R^{10}$ Chlor, Methyl oder Trifluormethyl bedeuten, oder der Molekülteil

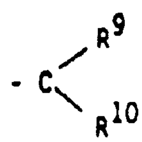

eine -C=$CR^{12}R^{13}$-Gruppe bedeutet, worin $R^{12}$ und $R^{13}$ jeweils für Wasserstoff, Methyl, Trifluormethyl, Fluor, Chlor oder Brom oder $R^{12}$ für Wasserstoff und $R^{13}$ für Fluor, Chlor oder Brom stehen, oder der Molekülteil

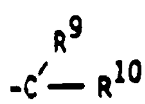

einen Drei- oder Vierring

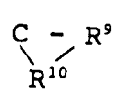

bedeutet, worin $R^9$ Sauerstoff oder eine $CR^{14}R^{15}$-Gruppe, worin die Gruppen $R^{14}$ und $R^{15}$ gleich oder verschieden sind und jeweils Wasserstoff, Fluor, Chlor oder Brom oder Methyl oder Ethyl, das gegebenenfalls durch 1 bis 3 Fluoratome substituiert ist und worin $R^{10}$ eine $CR^{14a}R^{15a}$ Gruppe oder eine $CR^{14a}R^{15a}CR^{16}R^{17}$-Gruppe, worin $R^{14a}$, $R^{15a}$ gleich oder verschieden sind und jeweils Wasserstoff, Fluor, Chlor oder Brom oder Methyl oder Ethyl, das gegebenenfalls durch 1 bis 5 Fluoratome substituiert ist, sowie $R^{16}$ und $R^{17}$ gleich oder verschieden sind und jeweils Wasserstoff, Fluor, Chlor oder Brom oder Methyl oder Ethyl, das gegebenenfalls durch 1 bis 5 Fluoratome substituiert ist; und $R^{5b}$ Wasserstoff oder $C_{1-3}$-Alkyl bedeuten; vorausgesetzt, daß $R^{2b}$ weder Propyl noch Butyl bedeutet und daß $R^{5a}$ weder tertiäres Butyl noch tertiäres Amyl bedeutet, wobei das Verfahren darin besteht, daß man

i) eine Verbindung der Formel (II):

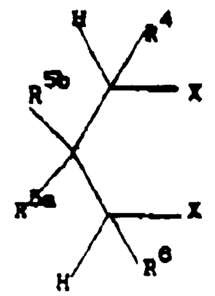

(II)

worin X SH bedeutet, mit einem geeigneten Aldehyd oder Keton der Formel

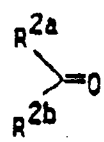

oder mit einem reaktiven Derivat davon, worin $R^{2a}$, $R^{2b}$, $R^4$, $R^{5a}$, $R^{5b}$ und $R^6$ wie oben angegeben definiert sind umsetzt und anschließend gegebenenfalls eines oder beide der Ringschwefelatome oxidiert,

(ii) wenn $R^{2a}$ Wasserstoff bedeutet, einen Dithiaborinandimethylsulfidkomplex einer Verbindung der Formel (II) mit einer Carbonsäure

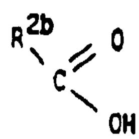

umsetzt, wobei diese Umsetzung in Anwesenheit eines Mittels wie z.B. Sn(II)-Chlorid in einem inerten Lösungsmittel wie einem Ether, zweckmäßig Tetrahydrofuran, bei einer nicht extremen Temperatur, zum Beispiel zwischen20° und 100° und zweckmäßig zwischen 10° und 30° erfolgt, und man anschließend gegebenenfalls eine Verbindung der Formel (I) mit Hilfe von dem Fachman bekannten Verfahren zu einer anderen Verbindung der Formel (I) umsetzt.

2. Verfahren zur Herstellung einer Verbindung der Formel (I) nach Anspruch 1, worin $R^{2b}$ eine in der 3-, 4- oder 5-Stellung durch jeweils aus Halogen, $C_{1-4}$-Halogenalkyl, $C_{1-4}$-Halogenalkoxy, $C_{1-4}$-Halogenalkylthio, Cyan ausgewählte Substituenten einfach bis dreifach substituierte Phenylgruppe oder eine Gruppe $(C{\equiv}C)_pR^{19}$ bedeutet, worin p 1 oder 2 und $R^{19}$ Wasserstoff, Brom, Chlor, Jod oder eine Gruppe $S(O)_qR^{20}$ bedeuten, worin q 0, 1 oder 2 und $R^{20}$ Trifluormethyl, Methyl oder Ethyl; oder $R^{19}$ eine gegebenenfalls durch $C_{1-4}$-Alkoxy, $C_{1-6}$-Alkoxyalkoxy, $C_{1-8}$-Acyloxy, Halogen oder Hydroxyl substituierte aliphatische Gruppe mit bis zu fünf Kohlenstoffatomen, oder $R^{19}$ eine $COR^{21}$-Gruppe, worin $R^{21}$ Wasserstoff, $C_{1-4}$-Alkoxy, $C_{1-4}$-Alkyl oder eine $NR^{22}R^{23}$-Gruppe, worin $R^{22}$ und $R^{23}$ unabhängig aus Wasserstoff, Methyl oder Ethyl ausgewählt sind; oder $R^{19}$ $SiR^{24}R^{25}R^{26}$, worin $R^{24}$ und $R^{25}$ gleich oder verschieden sind und jeweils $C_{1-4}$-aliphatische Gruppen und $R^{26}$ eine $C_{1-4}$-aliphatische Gruppe oder Phenyl bedeuten, vorausgesetzt, daß $R^{24}$, $R^{25}$ und $R^{26}$ insgesamt nicht mehr als 10 Kohlenstoffatome aufweisen und die Phenylgruppe gegebenenfalls in der 2 - und/oder 6-Stellung

durch Fluor oder Chlor weiter substituiert ist.

3. Verfahren zur Herstellung einer Verbindung der Formel (I) nach Anspruch 1, worin $R^{2b}$ eine Gruppe -A(C≡C)Z, worin A eine $C_{3-5}$-aliphatische Kette darstellt, die gegebenenfalls eine Doppelbindung und/oder ein Sauerstoffatom und/oder eine S(O)q-Gruppe aufweist, worin q 0, 1 oder 2 bedeutet, und gegebenenfalls durch Halogen, $C_{1-4}$-Alkyl, $C_{1-4}$-Halogenalkyl, $C_{1-4}$-Carbalkoxy oder Cyan substituiert ist, und Z Wasserstoff, $C_{1-5}$-Alkyl, $C_{1-3}$-Alkoxymethyl oder eine $SiR^{24}R^{25}R^{26}$-Gruppe bedeuten, worin $R^{24}$, $R^{25}$ und $R^{26}$ wie oben angegeben definiert sind.

4. Verfahren zur Herstellung einer Verbindung der Formel (I) nach Anspruch 1, worin $R^{2b}$ eine Gruppe -$BZ^{I}$, worin B eine Gruppe -$CH_2O$- oder $CH_2S(O)q$ darstellt, worin q 0, 1 oder 2, oder eine $C_{2-3}$-aliphatische Gruppe, die jeweils gegebenenfalls durch ein bis drei Halogenatome substituiert sind, sowie $Z^1$ durch drei $C_{1-4}$-Alkylgruppen substituiertes Silyl oder $Z^1$ eine Gruppe

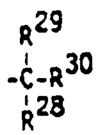

worin $R^{28}$, $R^{29}$ und $R^{30}$ gleich oder verschieden sind und jeweils unabhängig aus Halogen, Cyan, $C_{1-5}$-Carbalkoxy ausgewählt sind, oder eine gegebenenfalls durch Halogen, Cyan, $C_{1-5}$-Carbalkoxy, $C_{1-4}$-Alkoxy oder eine $S(O)qR^{31}$-Gruppe, worin q 0, 1 oder 2 und $R^{31}$ $C_{1-4}$-Alkyl ist, substituierte $C_{1-4}$-aliphatische Gruppe, oder $R^{28}$, $R^{29}$ und $R^{30}$ aus $C_{1-4}$-Alkoxy oder einer $S(O)_wR^{32}$-Gruppe ausgewählt sind, worin w 0, 1 oder 2 ist und $R^{32}$ gegebenenfalls durch Fluor substituiertes $C_{1-4}$-Alkyl bedeuten, oder $R^{28}$ und $R^{29}$ unter Bildung eines $C_{3-6}$-Cycloalkylrings verknüpft sind, oder einer der Reste $R^{28}$, $R^{29}$ und $R^{30}$ Wasserstoff bedeuten kann.

5. Verfahren zur Herstellung einer Verbindung der Formel (I) nach Anspruch 1, worin $R^{2b}$ eine Gruppe

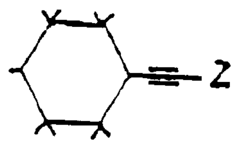

bedeutet, worin Z wie zuvor definiert ist.

6. Verfahren zur Herstellung einer Verbindung der Formel (I) nach einem der Ansprüche 1 bis 5, worin $R^{2a}$ Wasserstoff oder Methyl und $R^4$ und $R^6$ Wasserstoff bedeuten.

7. Verfahren zur Herstellung einer Verbindung der Formel (I) nach einem der Ansprüche 1 bis 6, worin $R^{5a}$ eine Isopropyl-, Isopropenyl-, Cyclopropyl-, Methylcyclopropyl-, Cyclobutyl, 1-Trifluormethylethyl- oder 1,1-Dimethyl-2,2,2-trifluorethylgruppe bedeutet.

8. Verfahren zur Herstellung einer Verbindung der Formel (I) nach Anspruch 7, worin $R^{5a}$ eine Isopropylgruppe bedeutet.

9. Verfahren zur Herstellung einer Verbindung der Formel (I) nach einem der Ansprüche 1 bis 8, ausgewählt aus der Gruppe:
cis/trans-2-(4-Bromphenyl)-5-isopropyl-1,3-dithian,
cis/trans-5-isopropyl-2-(4-trimethylsilylethinylphenyl)-1,3-dithian,
cis/trans-2-(4-Ethinylphenyl)-5-isopropyl-1,3-dithian,
trans-2-(4-Ethinylphenyl)-5-isopropyl-1,3-dithian,
cis/trans-2-(4-Ethinylphenyl)-5-isopropyl-2-methyl-1,3-dithian,
cis/trans-2-[4-(3-Acetoxyprop-1-inyl)phenyl]-5-isopropyl-1,3-dithian,
trans-2-[4-(3-Acetoxyprop-1-inyl)phenyl]-5-isopropyl-1,3-dithian,
trans-2-[4-(3-Benzoyloxyprop-1-inyl)phenyl]-5-isopropyl-1,3-dithian,
cis/trans-2-(trans-4-Ethinylcyclohexyl)-5-isopropyl-1,3-dithian,
cis/trans-2-(Hex-5-inyl)-5-isopropyl-1,3-dithian,
cis/trans-2-(trans-4-Ethinylcyclohexyl)-5-isopropyl-1,3-dithianoxid,
trans-2-(4-Bromphenyl)-5-isopropyl-1,3-dithian 1,3-dioxid,
2(e)-(4-Ethinylphenyl)-5(e)-isopropyl-1,3-dithian-1(e)-oxid,
cis-2(e)-(4-Ethinylphenyl)-5(a)-isopropyl-1,3-dithian,
trans-2(e)-(trans-4(e)-Ethinylcyclohexyl)-5(e)-isopropyl-1,3-dithian-1(e)-oxid,

trans-2(e)-[(E)-Hex-1-en-5-inyl)-5(e)-isopropyl-1,3-dithian,
cis/trans-5-isopropyl-2-methyl-2-(3,3,3-trichlorpropyl)-1,3-dithian,
cis/trans-2(e)-(4-Ethinylphenyl)-5-(1-trifluormethylethyl)-1,3-dithian,
trans-2(e)-(4-Ethinylphenyl)-5(e)-(1-trifluormethylethyl)-1,3-dithian,
trans-2(e)-(trans-4(e)-Ethinylcyclohexyl)-5(e)-(1-trifluormethylethyl)-1,3-dithian,
cis/trans-5(e)-(1,1-Dimethyl-2,2,2-trifluorethyl)-2-(4-trimethylsilylethinylphenyl)-1,3-dithian,
trans-5(e)-(1,1-Dimethyl-2,2,2-trifluorethyl)-2(e)-(4-ethinylphenyl)-1,3-dithian,
cis/trans-5(e)-(1,1-Dimethyl-2,2,2-trifluorethyl)-2-(4-ethinylphenyl)-1,3-dithian,
cis/trans-5-Cyclopropyl-2-(cis/trans-4-ethinylcyclohexyl)-1,3-dithian,
trans-5(e)-Cyclopropyl-2(e)-(4-ethinylphenyl)-1,3-dithian,
trans-5-Cyclobutyl-2-(4-ethinylphenyl)-1,3-dithian,
trans-2-(4-Ethinylphenyl)-5-(1-methylcyclopropyl)-1,3-dithian,
cis/trans-5-(1-Methylpropyl)-2-(4-ethinylphenyl)-1,3-dithian,
trans-5(e)-(1-Methylpropyl)-2(e)-(4-trimethylsilylethinylphenyl)-1,3-dithian.

10. Insektizide oder akarazide Zusammensetzung aus einer gemäß einem der Ansprüche 1-8 definierten Verbindung der Formel (I) in einem Gemisch mit einem Träger oder Verdünnungsmittel.

11. Synergisierte schädlingsbekämpfende Zusammensetzung mit einer gemäß einem der Ansprüche 1-8 definierten Verbindung der Formel (I), einem Synergisten für die Verbindung der Formel (I) und einem Träger oder Verdünnungsmittel.

12. Gemisch aus einer gemäß einem der Ansprüche 1-8 definierten Verbindung der Formel (I) und einer weiteren schädlingsbekämpfenden Verbindung.

13. Verfahren zur Schädlingsbekämpfung, das darin besteht, daß man eine zur Schädlingsbekämpfung wirksame Menge einer Verbindung nach einem der Ansprüche 1-8 oder einer Zusammensetzung oder eines Gemisches nach einem der Ansprüche 10-12 auf die Schädlinge oder eine vom Schädlingsbefall bedrohte Fläche einwirken läßt.

14. Verbindung gemäß einem der Ansprüche 1-9 oder Zusammensetzung nach einem der Ansprüche 10-12 zur Verwendung in einem Verfahren zur chirurgischen oder therapeutischen Behandlung des menschlichen oder tierischen Körpers oder in einem Diagnostizierverfahren, das am menschlichen oder tierischen Körper vorgenommen wird.

## Revendications

### Revendications pour les Etats contractants suivants : AT, BE, CH, DE, FR, GB, IT, LI, LU, NL, SE

1. Composé de formule (I) :

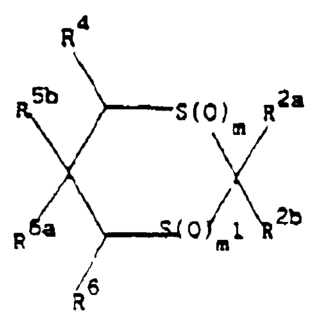

(I)

qui contient entre 9 et 27 atomes de carbone et dans lequel m et $m^1$ sont choisis indépendamment parmi 0, 1 et 2; $R^{2a}$ est un hydrogène, un groupe méthyle ou éthyle; $R^{2b}$ est un groupe éthynyle ou contient entre 3 et 18 atomes de carbone et est un groupe $R^7$, dans lequel $R^7$ est un groupe hydrocarbyle non aromatique en $C_1$-$C_{13}$, éventuellement substitué par un groupe cyano ou un groupe carbalcoxy en $C_1$-$C_4$ et/ou par un ou deux groupes hydroxy et/ou par un à cinq atomes d'halogène qui sont identiques ou différents et/ou par un à trois groupes $R^8$ qui sont identiques ou différents et qui contiennent chacun un à quatre hétéroatomes, qui sont identiques ou différents et sont choisis parmi l'oxygène, le soufre, l'azote et le silicium, 1 à 10 atomes de carbone et éventuellement 1 à 6 atomes de fluor ou de chlore, ou $R^{2b}$ est un noyau aromatique à 6 chaînons substitué par un groupe cyano et/ou par un à trois groupes $R^{8a}$ et/ou par un groupe -C=CH, -C=C-$R^{7a}$ ou -C=C-halogéno et/ou

par un à cinq atomes d'halogène et/ou par un à trois groupes halogénoalkyle en $C_1$-$C_4$, où $R^{7a}$ et $R^{8a}$ sont des groupes $R^7$ et $R^8$ tels que définis ci-dessus; $R^4$ et $R^6$ sont identiques ou différents et sont choisis parmi des atomes d'hydrogène, des groupes méthyle, trifluorométhyle ou cyano; $R^{5a}$ est un groupe

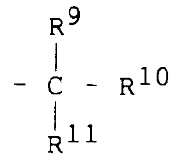

dans lequel $R^{11}$ est un hydrogène, un groupe méthyle, trifluorométhyle, iodo, fluoro, chloro ou bromo, $R^9$ est un groupe méthyle, éthyle, chloro, bromo, méthoxy, cyano, nitro, méthoxyméthyle, carbalcoxy en $C_1$-$C_4$ ou trifluorométhyle, $R^{10}$ est un groupe chloro, méthyle ou trifluorométhyle, ou bien le groupement

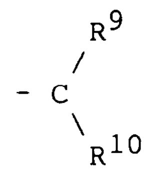

est un groupe -C=CR$^{12}$R$^{13}$, dans lequel $R^{12}$ et $R^{13}$ sont tous deux des atomes d'hydrogène, des groupes méthyle, trifluorométhyle, fluoro, chloro ou bromo, ou $R^{12}$ est un hydrogène et $R^{13}$ est un groupe fluoro, chloro ou bromo, ou bien le groupement

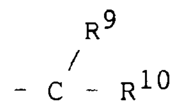

est un noyau

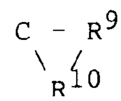

à trois ou quatre chaînons, dans lequel $R^9$ est un oxygène ou un groupe CR$^{14}$R$^{15}$, dans lequel les groupes $R^{14}$ et $R^{15}$ sont identiques ou différents et chacun est un hydrogène, un groupe fluoro, chloro ou bromo, ou méthyle ou éthyle éventuellement substitué par 1 à 3 atomes de fluor, et dans lequel $R^{10}$ est un groupe CR$^{14a}$R$^{15a}$ ou un groupe CR$^{14a}$R$^{15a}$CR$^{16}$R$^{17}$, dans lesquels les groupes $R^{14a}$ et $R^{15a}$ sont identiques ou différents et chacun est un hydrogène, un groupe fluoro, chloro ou bromo, ou méthyle ou éthyle éventuellement substitué par 1 à 5 atomes de fluor, et $R^{16}$ et $R^{17}$ sont identiques ou différents et chacun est un hydrogène, un groupe fluoro, chloro ou bromo, ou méthyle ou éthyle éventuellement substitué par 1 à 5 atomes de fluor; et $R^{5b}$ est un hydrogène ou un groupe alkyle en $C_1$-$C_3$; à condition que $R^{2b}$ ne soit ni un groupe propyle ni un groupe butyle et que $R^{5a}$ ne soit ni un groupe t-butyle ni un groupe t-amyle.

2. Composé de formule (I) selon la revendication 1, dans lequel $R^{2b}$ est un groupe phényle substitué en position 3, 4 ou 5 par un à trois substituants choisis chacun parmi un groupe halogéno, halogénoalkyle en $C_1$-$C_4$, halogénoalcoxy en $C_1$-$C_4$, halogénoalkylthio en $C_1$-$C_4$, cyano ou un groupe $(C{=}C)_pR^{19}$, dans lequel p est égal à 1 ou 2 et $R^{19}$ est un hydrogène, un groupe bromo, chloro, iodo ou un groupe $S(O)_qR^{20}$, dans lequel q est égal à 0, 1 ou 2 et $R^{20}$ est un groupe trifluorométhyle, méthyle ou éthyle; ou $R^{19}$ est un groupe aliphatique contenant jusqu'à cinq atomes de carbone, éventuellement substitué par un groupe alcoxy en $C_1$-$C_4$, alcoxyalcoxy en $C_1$-$C_6$, acyloxy en $C_1$-$C_8$, halogéno ou hydroxy, ou $R^{19}$ est un groupe COR$^{21}$, dans lequel $R^{21}$ est un hydrogène, un groupe alcoxy en $C_1$-$C_4$, alkyle en $C_1$-$C_4$ ou un groupe NR$^{22}$R$^{23}$, dans lequel $R^{22}$ et $R^{23}$ sont choisis indépendamment parmi un hydrogène, un groupe méthyle ou éthyle; ou $R^{19}$ est SiR$^{24}$, $R^{25}$, $R^{26}$, dans lequel $R^{24}$ et $R^{25}$ sont identiques ou différents et sont chacun des groupes aliphatiques en $C_1$-$C_4$ et $R^{26}$ est un groupe aliphatique en $C_1$-$C_4$ ou phényle, à condition que $R^{24}$, $R^{25}$ et $R^{26}$ ne contiennent pas plus de 10 atomes de carbone au total, et le groupe phényle est aussi éventuellement substitué en positions 2 et/ou 6 par un groupe fluoro ou chloro.

3. Composé de formule (I) selon la revendication 1, dans lequel $R^{2b}$ est un groupe -A(C=C)Z, dans lequel

A est une chaîne aliphatique en $C_3$-$C_5$ contenant éventuellement une double liaison et/ou un atome d'oxygène et/ou un groupe $S(O)_q$, dans lequel q est égal à 0, 1 ou 2, éventuellement substitué par un groupe halogéno, alkyle en $C_1$-$C_4$, halogénoalkyle en $C_1$-$C_4$, carbalcoxy en $C_1$-$C_4$ ou cyano, et Z est un hydrogène, un groupe alkyle en $C_1$-$C_5$, alcoxy(en $C_1$-$C_3$)méthyle ou un groupe $SiR^{24}$, $R^{25}$, $R^{26}$, dans lequel $R^{24}$, $R^{25}$ et $R^{26}$ sont tels que définis ci-dessus.

4. Composé de formule (I) selon la revendication 1, dans lequel $R^{2b}$ est un groupe $-BZ^1$, dans lequel B est un groupe $-CH_2O-$ ou $CH_2S(O)_q$, dans lequel q est égal à 0, 1 ou 2, ou un groupe aliphatique en $C_2$-$C_3$, chacun pouvant être éventuellement substitué par un à trois atomes d'halogène, et $Z^1$ est un groupe silyle substitué par trois groupes alkyle en $C_1$-$C_4$, ou $Z^1$ est un groupe

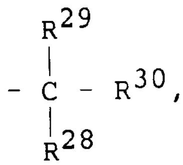

dans lequel $R^{28}$, $R^{29}$ et $R^{30}$ sont identiques ou différents et sont chacun choisis indépendamment parmi les groupes halogéno, cyano, carbalcoxy en $C_1$-$C_5$, ou un groupe aliphatique en $C_1$-$C_4$ éventuellement substitué par un groupe halogéno, cyano, carbalcoxy en $C_1$-$C_5$, alcoxy en $C_1$-$C_4$ ou un groupe $S(O)_qR^{31}$, dans lequel q est égal à 0, 1 ou 2 et $R^{31}$ est un groupe alkyle en $C_1$-$C_4$, ou $R^{28}$, $R^{29}$ et $R^{30}$ sont choisis parmi un groupe alcoxy en $C_1$-$C_4$ ou un groupe $S(O)_wR^{32}$, dans lequel w est égal à 0, 1 ou 2, et $R^{32}$ est un groupe alkyle en $C_1$-$C_4$ éventuellement substitué par un groupe fluoro, ou $R^{28}$ et $R^{29}$ sont liés pour former un noyau cycloalkyle en $C_3$-$C_6$, ou l'un des groupes $R^{28}$, $R^{29}$ et $R^{30}$ peut être un hydrogène. 5. Composé de formule (I) selon la revendication 1, dans lequel $R^{2b}$ est un groupe

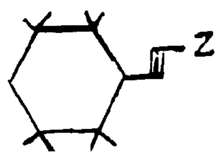

dans lequel Z est tel que défini ci-dessus.

6. Composé de formule (I) selon l'une quelconque des revendications 1 à 5, dans lequel $R^{2a}$ est un hydrogène ou un groupe méthyle, et $R^4$ et $R^6$ sont des atomes d'hydrogène.

7. Composé de formule (I) selon l'une quelconque des revendications 1 à 6, dans lequel $R^{5a}$ est un groupe isopropyle, isopropényle, cyclopropyle, méthylcyclopropyle, cyclobutyle, 1-trifluorométhyléthyle ou 1,1-diméthyl-2,2,2-trifluoroéthyle.

8. Composé de formule (I) selon la revendication 7, dans lequel $R^{5a}$ est un groupe isopropyle.

9. Composé de formule (I) selon la revendication 1, choisi dans le groupe comprenant :

le cis/trans-2-(4-bromophényl)-5-isopropyl-1,3-dithiane,
le cis/trans-5-isopropyl-2-(4-triméthylsilyéthynylphényl)-1,3-dithiane,
le cis/trans-2-(4-éthynylphényl)-5-isopropyl-1,3-dithiane,
le trans-2-(4-éthynylphényl)-5-isopropyl-1,3-dithiane,
le cis/trans-2-(4-éthynylphényl)-5-isopropyl-2-méthyl-1,3-dithiane,
le cis/trans-2-[4-(3-acétoxyprop-1-ynyl)phényl]-5-isopropyl-1,3-dithiane,
le trans-2-[4-(3-acétoxyprop-1-ynyl)phényl]-5-isopropyl-1,3-dithiane,
le trans-2-[4-(3-benzoyloxyprop-1-ynyl)phényl]-5-isopropyl-1,3-dithiane,
le cis/trans-2-(trans-4-éthynylcyclohexyl)-5-isopropyl-1,3-dithiane,
le cis/trans-2-(hex-5-ynyl)-5-isopropyl-1,3-dithiane,
l'oxyde de cis/trans-2-(trans-4-éthynylcyclohexyl)-5-isopropyl-1,3-dithiane,
le 1,3-dioxyde de trans-2-(4-bromophényl)-5-isopropyl-1,3-dithiane,
le 1(e)-oxyde de 2(e)-(4-éthynylphényl)-5(e)-isopropyl-1,3-dithiane,
le cis-2(e)-(4-éthynylphényl)-5(a)-isopropyl-1,3-dithiane,
le 1(e)-oxyde de trans-2(e)-(trans-4(e)-éthynylcyclohexyl)-5(e)-isopropyl-1,3-dithiane,
le trans-2(e)-[(E)-hex-1-ène-5-ynyl)-5(e)-isopropyl-1,3-dithiane,
le cis/trans-5-isopropyl-2-méthyl-2-(3,3,3-trichloropropyl)-1,3-dithiane,
le cis/trans-2(e)-(4-éthynylphényl)-5-(1-trifluorométhyléthyl)-1,3-dithiane,
le trans-2(e)-(4-éthynylphényl)-5(e)-(1-trifluorométhyléthyl)-1,3-dithiane,

le trans-2(e)-(trans-4(e)-éthynylcyclohexyl)-5(e)-(1-trifluorométhyléthyl)-1,3-dithiane,
le cis/trans-5(e)-(1,1-diméthyl-2,2,2-trifluoroéthyl)-2-(4-triméthylsilyéthynylphényl)-1,3-dithiane,
le trans-5(e)-(1,1-diméthyl-2,2,2-trifluoroéthyl)-2(e)-(4-éthynylphényl)-1,3-dithiane,
le cis/trans-5(e)-(1,1-diméthyl-2,2,2-trifluoroéthyl)-2(4-éthynylphényl)-1,3-dithiane,
le cis/trans-5-cyclopropyl-2-(cis/trans-4-éthynylcyclohexyl)-1,3-dithiane,
le trans-5(e)-cyclopropyl-2(e)-(4-éthynylphényl)-1,3-dithiane,
le trans-5-cyclobutyl-2-(4-éthynylphényl)-1,3-dithiane,
le trans-2-(4-éthynylphényl)-5-(1-méthylcyclopropyl)-1,3-dithiane,
le cis/trans-5-(1-méthylpropyl)-2-(4-éthynylphényl)-1,3-dithiane,
le trans-5(e)-(1-méthylpropyl)-2(e)-(4-triméthylsilyéthynylphényl)-1,3-dithiane.

10. Procédé de préparation d'un composé de formule (I) selon l'une quelconque des revendications 1 à 9, qui comprend

i) la réaction d'un composé de formule (II) :

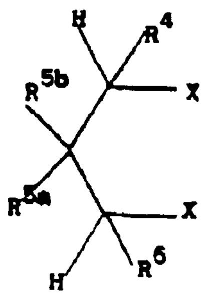

(II)

dans laquelle X est SH, avec un aldéhyde ou une cétone convenable de formule

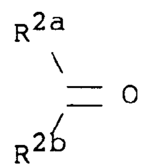

ou un de ses dérivés réactifs, où $R^{2a}$ $R^{2b}$ $R^4$ $R^{5a}$ $R^{5b}$ et $R^6$ sont tels que définis ci-dessus et, éventuellement, par la suite, l'oxydation d'un des atomes de soufre du noyau ou des deux,

(ii) lorsque $R^{2a}$ est un hydrogène, la réaction d'un complexe dithiaborinane-sulfure de diméthyle d'un composé de formule (II) avec un acide carboxylique

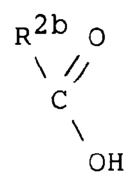

.

Cette réaction est réalisée en présence d'un agent tel que le chlorure stanneux, dans un solvant inerte tel qu'un éther, avantageusement le tétrahydrofuranne, à une température non extrême, par exemple entre -20° et 100° et avantageusement entre 10° et 30°,

puis, par la suite, éventuellement, la transformation d'un composé de formule (I) en un autre composé de formule (I), par des procédés bien connus de l'homme de métier.

11. Composition insecticide ou acaricide comprenant un composé de formule (I) selon l'une quelconque des revendications 1-9 en mélange avec un véhicule ou un diluant.

12. Composition pesticide, rendue synergique, comprenant un composé de formule (I) selon l'une quelconque des revendications 1-9, un agent de synergie pour le composé de formule (I) et un véhicule ou un diluant.

13. Mélange d'un composé de formule (I) selon l'une quelconque des revendications 1-9 et d'un autre composé pesticide.

14. Procédé de lutte contre les nuisibles, comprenant l'application au nuisible, ou à un environnement susceptible d'être infesté par les nuisibles, d'une quantité efficace en tant que pesticide d'un composé selon l'une quelconque des revendications 1-9 ou d'une composition ou d'un mélange selon l'une quelconque des revendications 11-13.

15. Composé selon l'une quelconque des revendications 1-9 ou composition selon l'une quelconque des revendications 11-13 utilisable dans un procédé de chirurgie ou de thérapeutique pratiqué sur le corps humain ou animal ou dans un procédé de diagnostic pratiqué sur le corps humain ou animal.

**Revendications pour les Etats suivants : ES, GR**

1. Procédé de préparation d'un composé de formule (I) :

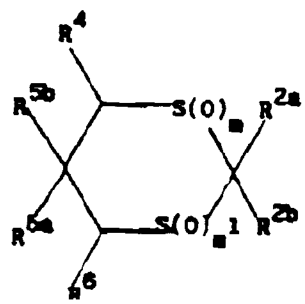

(I)

qui contient entre 9 et 27 atomes de carbone et dans lequel m et $m^1$ sont choisis indépendamment parmi 0, 1 et 2; $R^{2a}$ est un hydrogène, un groupe méthyle ou éthyle; $R^{2b}$ est un groupe éthynyle ou contient entre 3 et 18 atomes de carbone et est un groupe $R^7$, dans lequel $R^7$ est un groupe hydrocarbyle non aromatique en $C_1$-$C_{13}$, éventuellement substitué par un groupe cyano ou un groupe carbalcoxy en $C_1$-$C_4$ et/ou par un ou deux groupes hydroxy et/ou par un à cinq atomes d'halogène qui sont identiques ou différents et/ou par un à trois groupes $R^8$ qui sont identiques ou différents et qui contiennent chacun un à quatre hétéroatomes, qui sont identiques ou différents et sont choisis parmi l'oxygène, le soufre, l'azote et le silicium, 1 à 10 atomes de carbone et éventuellement 1 à 6 atomes de fluor ou de chlore, ou $R^{2b}$ est un noyau aromatique à 6 chaînons substitué par un groupe cyano et/ou par un à trois groupes $R^{8a}$ et/ou par un groupe -C=CH, -C=C-$R^{7a}$ ou -C=C-halogéno et/ou par un à cinq atomes d'halogène et/ou par un à trois groupes halogénoalkyle en $C_1$-$C_4$, où $R^{7a}$ et $R^{8a}$ sont des groupes $R^7$ et $R^8$ tels que définis ci-dessus; $R^4$ et $R^6$ sont identiques ou différents et sont choisis parmi des atomes d'hydrogène, des groupes méthyle, trifluorométhyle ou cyano; $R^{5a}$ est un groupe

```
    R9
    |
  - C - R10
    |
    R11
```

dans lequel $R^{11}$ est un hydrogène, un groupe méthyle, trifluorométhyle, iodo, fluoro, chloro ou bromo, $R^9$ est un groupe méthyle, éthyle, chloro, bromo, méthoxy, cyano, nitro, méthoxyméthyle, carbalcoxy en $C_1$-$C_4$ ou trifluorométhyle, $R^{10}$ est un groupe chloro, méthyle ou trifluorométhyle, ou bien le groupement

```
      R9
     /
  - C
     \
      R10
```

est un groupe -C=$CR^{12}R^{13}$, dans lequel $R^{12}$ et $R^{13}$ sont tous deux des atomes d'hydrogène, des groupes méthyle, trifluorométhyle, fluoro, chloro ou bromo, ou $R^{12}$ est un hydrogène et $R^{13}$ est un groupe fluoro, chloro ou bromo, ou bien le groupement

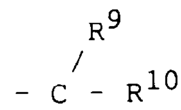

est un noyau

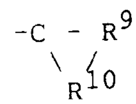

à trois ou quatre chaînons, dans lequel $R^9$ est un oxygène ou un groupe $CR^{14}R^{15}$, dans lequel les groupes $R^{14}$ et $R^{15}$ sont identiques ou différents et chacun est un hydrogène, un groupe fluoro, chloro ou bromo, ou méthyle ou éthyle éventuellement substitué par 1 à 3 atomes de fluor, et dans lequel $R^{10}$ est un groupe $CR^{14a}R^{15a}$ ou un groupe $CR^{14a}R^{15a}CR^{16}R^{17}$, dans lesquels les groupes $R^{14a}$ et $R^{15a}$ sont identiques ou différents et chacun est un hydrogène, un groupe fluoro, chloro ou bromo, ou méthyle ou éthyle éventuellement substitué par 1 à 5 atomes de fluor, et $R^{16}$ et $R^{17}$ sont identiques ou différents et chacun est un hydrogène, un groupe fluoro, chloro ou bromo, ou méthyle ou éthyle éventuellement substitué par 1 à 5 atomes de fluor; et $R^{5b}$ est un hydrogène ou un groupe alkyle en $C_1$-$C_3$; à condition que $R^{2b}$ ne soit ni un groupe propyle ni un groupe butyle et que $R^{5a}$ ne soit ni un groupe t-butyle ni un groupe t-amyle, le procédé comprenant :

i) la réaction d'un composé de formule (II)

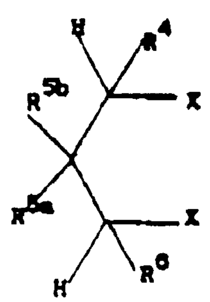

(II)

dans laquelle X est SH, avec un aldéhyde ou une cétone convenable de formule

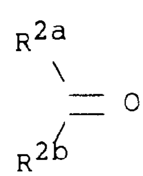

ou un de ses dérivés réactifs, où $R^{2a}$ $R^{2b}$ $R^4$ $R^{5a}$ $R^{5b}$ et $R^6$ sont tels que définis ci-dessus et, éventuellement, par la suite, l'oxydation d'un des atomes de soufre du noyau ou des deux,

(ii) lorsque $R^{2a}$ est un hydrogène, la réaction d'un complexe dithiaborinane-sulfure de diméthyle d'un composé de formule (II) avec un acide carboxylique

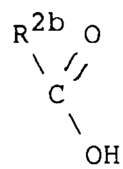

.

Cette réaction est réalisée en présence d'un agent tel que le chlorure stanneux, dans un solvant inerte tel qu'un éther, avantageusement le tétrahydrofuranne, à une température non extrême, par exemple entre -20° et 100° et avantageusement entre 10° et 30°, puis, par la suite, éventuellement, la transformation d'un composé de formule (I) en un autre composé de formule (I), par des procédés bien connus de l'homme de métier.

2. Procédé de préparation d'un composé de formule (I) selon la revendication 1, dans lequel $R^{2b}$ est un groupe phényle substitué en position 3, 4 ou 5 par un à trois substituants choisis chacun parmi un groupe halogéno, halogénoalkyle en $C_1$-$C_4$, halogénoalcoxy en $C_1$-$C_4$, halogénoalkylthio en $C_1$-$C_4$, cyano ou un groupe $(C{=}C)_pR^{19}$, dans lequel p est égal à 1 ou 2 et $R^{19}$ est un hydrogène, un groupe bromon chloro, iodo ou un groupe $S(O)_qR^{20}$, dans lequel q est égal à 0, ou 2 et $R^{20}$ est un groupe trifluorométhyle, méthyle ou éthyle; ou $R^{19}$ est un groupe aliphétique contenant jusqu'à cinq atomes de carbone, éventuellement substitué par un groupe alcoxy en $C_1$-$C_4$, alcoxyalcoxy en $C_1$-$C_6$, acyloxy en $C_1$-$C_8$, halogéno ou hydroxy, ou $R^{19}$ est un groupe $COR^{21}$, dans lequel $R^{21}$ est un hydrogène, un groupe alcoxy en $C_1$-$C_4$, alkyle en $C_1$-$C_4$ ou un groupe $NR^{22}R^{23}$, dans lequel $R^{22}$ et $R^{23}$ sont choisis indépendamment parmi un hydrogène, un groupe méthyle ou éthyle; ou $R^{19}$ est $SiR^{24}$, $R^{25}$, $R^{26}$, dans lequel $R^{24}$ et $R^{25}$ sont identiques ou différents et sont chacun des groupes aliphatiques en $C_1$-$C_4$ et $R^{26}$ est un groupe aliphatique en $C_1$-$C_4$ ou phényle, à condition que $R^{24}$, $R^{25}$ et $R^{26}$ ne contiennent pas plus de 10 atomes de carbone au total, et le groupe phényl est aussi éventuellement substitué en position 2 et/ou 6 par un groupe fluoro ou chloro.

3. Procédé de préparation d'un composé de formule (I) selon la revendication 1, dans lequel $R^{2b}$ est un groupe -A(C=C)Z, dans lequel A est une chaîne aliphatique en $C_3$-$C_5$ contenant éventuellement une double liaison et/ou un atome d'oxygène et/ou un groupe $S(O)_q$, dans lequel q est égal à 0, 1 ou 2, éventuellement substitué par un groupe halogéno, alkyle en $C_1$-$C_4$, halogénoalkyle en $C_1$-$C_4$, $C_4$, carbalcoxy en $C_1$-$C_4$ ou cyano, et Z est un hydrogène, un groupe alkyle en $C_1$-$C_5$, alcoxy(en $C_1$-$C_3$) méthyle ou un groupe $SiR^{24}$, $R^{25}$, $R^{26}$, dans lequel $R^{24}$, $R^{25}$ et $R^{26}$ sont tels que définis ci-dessus.

4. Procédé de préparation d'un composé de formule (I) selon la revendication 1, dans lequel $R^{2b}$ est un groupe $-BZ^1$, dans lequel B est un groupe $-CH_2$-O- ou $CH_2S(O)_q$, dans lequel q est égal à 0, 1 ou 2, ou un groupe aliphatique en $C_2$-$C_3$, chacun pouvant être éventuellement substitué par un à trois atomes d'halogène, et $Z^1$ est un groupe silyle substitué par trois groupes alkyle en $C_1$-$C_4$, ou $Z^1$ est un groupe

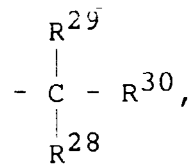

dans lequel $R^{28}$, $R^{29}$ et $R^{30}$ sont identiques ou différents et sont chacun choisis indépendamment parmi les groupes halogéno, cyano, carbalcoxy en $C_1$-$C_5$, ou un groupe aliphatique en $C_1$-$C_4$ éventuellement substitué par un groupe halogéno, cyano, carbalcoxy en $C_1$-$C_5$, alcoxy en $C_1$-$C_4$ ou un groupe $S(O)_qR^{31}$, dans lequel q est égal à 0, 1 ou 2, et $R^{31}$ est un groupe alkyle en $C_1$-$C_4$, ou $R^{28}$, $R^{29}$ et $R^{30}$ sont choisis parmi un groupe alcoxy en $C_1$-$C_4$ ou un groupe $S(O)_wR^{32}$, dans lequel w est égal à 0, 1 ou 2, et $R^{32}$ est un groupe alkyle en $C_1$-$C_4$ éventuellement substitué par un groupe fluoro, ou $R^{28}$ et $R^{29}$ sont liés pour former un noyau cycloalkyle en $C_3$-$C_6$, ou l'un des groupes $R^{28}$, $R^{29}$ et $R^{30}$ peut être un hydrogène.

5. Procédé de préparation d'un composé de formule (I) selon la revendication 1, dans lequel $R^{2b}$ est un groupe

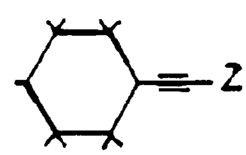

dans lequel Z est tel que défini ci-dessus.

6. Procédé de préparation d'un composé de formule (I) selon l'une quelconque des revendications 1 à 5, dans lequel $R^{2a}$ est un hydrogène ou un groupe méthyle, et $R^4$ et $R^6$ sont des atomes d'hydrogène.

7. Procédé de préparation d'un composé de formule (I) selon l'une quelconque des revendications 1 à 6, dans lequel $R^{5a}$ est un groupe isopropyle, isopropényle, cyclopropyle, méthylcyclopropyle, cyclobutyle, 1-trifluorométhyléthyle ou 1,1-diméthyl-2,2,2-trifluoroéthyle.

8. Procédé de préparation d'un composé de formule (I) selon la revendication 7, dans lequel $R^{5a}$ est un groupe isopropyle.

9. Procédé de préparation d'un composé de formule (I) selon l'une quelconque des revendications 1 à 8, choisi dans le groupe comprenant :

le cis/trans-2-(4-bromophényl)-5-isopropyl-1,3-dithiane,

le cis/trans-5-isopropyl-2-(4-triméthylsilyéthynylphényl)-1,3-dithiane,
le cis/trans-2-(4-éthynylphényl)-5-isopropyl-1,3-dithiane,
le trans-2-(4-éthynylphényl)-5-isopropyl-1,3-dithiane,
le cis/trans-2-(4-éthynylphényl)-5-isopropyl-2-méthyl-1,3-dithiane,
le cis/trans-2-[4-(3-acétoxyprop-1-ynyl)phényl]-5-isopropyl-1,3-dithiane,
le trans-2-[4-(3-acétoxyprop-1-ynyl)phényl]-5-isopropyl-1,3-dithiane,
le trans-2-[4-(3-benzoyloxyprop-1-ynyl)phényl]-5-isopropyl-1,3-dithiane,
le cis/trans-2-(trans-4-éthynylcyclohexyl)-5-isopropyl-1,3-dithiane,
le cis/trans-2-(hex-5-ynyl)-5-isopropyl-1,3-dithiane, l'oxyde de cis/trans-2-(trans-4-éthynylcyclohexyl)-5-isopropyl-1,3-dithiane,
le 1,3-dioxyde de trans-2-(4-bromophényl)-5-isopropyl-1,3-dithiane,
le 1(e)-oxyde de 2(e)-(4-éthynylphényl)-5(e)-isopropyl-1,3-dithiane,
le cis-2(e)-(4-éthynylphényl)-5(a)-isopropyl-1,3-dithiane,
le 1(e)-oxyde de trans-2(e)-(trans-4(e)-éthynylcyclohexyl)-5(e)-isopropyl-1,3-dithiane,
le trans-2(e)-[(E)-hex-1-ène-5-ynyl)-5(e)-isopropyl-1,3-dithiane,
le cis/trans-5-isopropyl-2-méthyl-2-(3,3,3-trichloropropyl)-1,3-dithiane,
le cis/trans-2(e)-(4-éthynylphényl)-5-(1-trifluorométhyléthyl)-1,3-dithiane,
le trans-2(e)-(4-éthynylphényl)-5(e)-(1-trifluorométhyléthyl)-1,3-dithiane,
le trans-2(e)-(trans-4(e)-éthynylcyclohexyl)-5(e)-(1-trifluorométhyléthyl)-1,3-dithiane,
le cis/trans-5(e)-(1,1-diméthyl-2,2,2-trifluoroéthyl)-2-(4-triméthylsilyléthynylphényl)-1,3-dithiane,
le trans-5(e)-(1,1-diméthyl-2,2,2-trifluoroéthyl)-2(e)-(4-éthynylphényl)-1,3-dithiane,
le cis/trans-5(e)-(1,1-diméthyl-2,2,2-trifluoroéthyl)-2-(4-éthynylphényl)-1,3-dithiane,
le cis/trans-5-cyclopropyl-2-(cis/trans-4-éthynylcyclohexyl)-1,3-dithiane,
le trans-5(e)-cyclopropyl-2(e)-(4-éthynylphényl)-1,3-dithiane,
le trans-5-cyclobutyl-2-(4-éthynylphényl)-1,3-dithiane,
le trans-2-(4-éthynylphényl)-5-(1-méthylcyclopropyl)-1,3-dithiane,
le cis/trans-5-(1-méthylpropyl)-2-(4-éthynylphènyl)-1,3-dithiane,
le trans-5(e)-(1-méthylpropyl)-2(e)-(4-triméthylsilyéthynylphényl)-1,3-dithiane.

10. Composition insecticide ou acaricide comprenant un composé de formule (I) selon l'une quelconque des revendications 1-8 en mélange avec un véhicule ou un diluant.

11. Composition pesticide, rendue synergique, comprenant un composé de formule (I) selon l'une quelconque des revendications 1-8, un agent de synergie pour le composé de formule (I) et un véhicule ou un diluant.

12. Mélange d'un composé de formule (I) selon l'une quelconque des revendications 1-8 et d'un autre composé pesticide.

13. Procédé de lutte contre les nuisibles, comprenant l'application au nuisible, ou à un environnement susceptible d'être infesté par les nuisibles, d'une quantité efficace en tant que pesticide d'un composé selon l'une quelconque des revendications 1-8 ou d'une composition ou d'un mélange selon l'une quelconque des revendications 10-12.

14. Composé selon l'une quelconque des revendications 1-9 ou composition selon l'une quelconque des revendications 10-12 utilisable dans un procédé de chirurgie ou de thérapeutique pratiqué sur le corps humain ou animal ou dans un procédé de diagnostic pratiqué sur le corps humain ou animal.